# EUROPEAN PATENT APPLICATION

(11) **EP 4 715 365 A1**
(43) Date of publication of application: **25.03.2026**
(21) Application number: 25202761.0
(22) Date of filing: 17.09.2025
(51) Int. Cl.: G01N 15/14, G01N 15/1429, G06V 20/69

(54) **SAMPLE ANALYZER AND SAMPLE ANALYSIS METHOD**

(30) Priority: 19.09.2024 JP 2024162471
(71) Applicant: Juntendo Educational Foundation, Tokyo 113-8421 (JP); SYSMEX CORPORATION, Kobe-shi Hyogo 651-0073 (JP)
(72) Inventor: SUZUKI, Kohjin, Hyogo 651-0073 (JP); TAKAKU, Tomoiku, Tokyo 113-8421 (JP)
(74) Representative: Becker, Eberhard

(57) **Abstract**

Disclosed is a specimen analysis apparatus (1A, 1B, 1C, 1) comprising:
a measurement unit (20) configured to acquire optical information of cells contained in a specimen by irradiating the cells with a plurality of diffracted lights generated by causing light to be incident on a diffractive optical element (215); and
an analysis unit (30) configured to analyze the optical information obtained by the measurement unit (20) using an artificial intelligence algorithm to acquire first information on leukemic cells contained in the specimen.

## Description

### CROSS REFERENCE TO RELATED APPLICATIONS

This application claims priority from prior Japanese Patent Application No. 2024-162471, filed on September 19, 2024, entitled "SAMPLE ANALYZER, SAMPLE ANALYSIS METHOD, AND METHOD", the entire content of which is incorporated herein by reference.

### FIELD OF THE INVENTION

The present invention relates to a sample analyzer and the like.

Chronic myeloid leukemia (CML) is a type of leukemia caused by abnormalities in pluripotent hematopoietic stem cells, and is characterized by the Philadelphia chromosome formed by t(9;22)(q34;q11). In CML, the BCR::ABL1 tyrosine kinase (TK) encoded by the BCR::ABL1 fusion gene on the Philadelphia chromosome is constitutively activated, is involved in the proliferation of leukemia cells, and progresses through three phases. The three phases are: a chronic phase with few subjective symptoms (lasting 3-5 years), an accelerated phase in which granulocyte differentiation abnormalities progress (lasting 3-9 months), and a blast crisis phase in which undifferentiated blasts increase and the disease resembles acute leukemia (lasting 3-6 months), ultimately becoming fatal.

### BACKGROUND OF THE INVENTION

Non-Patent Document 1: Jabbour E, Kantarjian H. Chronic myeloid leukemia: 2020 update on diagnosis, therapy and monitoring (Am J Hematol. 2020 Jun;95(6):691-709. doi: 10.1002/ajh.25792. Epub 2020 Apr 10. PMID: 32239758) discloses a method for diagnosing CML based on blood cell counting tests that detect an increase in leukocytes, particularly characteristic increases in neutrophils, eosinophils, and basophils.

### SUMMARY OF THE INVENTION

Chronic myeloid leukemia (CML) is a disease whose prognosis has been dramatically improved by the advent of tyrosine kinase inhibitors (TKIs), and it is considered that early detection further enhances therapeutic efficacy.
Although increases of leukocytes and basophils in peripheral blood are observed in CML, in actual clinical practice, CML is suspected and diagnosed based on abnormalities in leukocyte or basophil counts. Therefore, as disclosed in Non-Patent Document 1, it is difficult to screen patients in the early stage of onset before abnormalities in blood cell counts appear by conventional blood cell counting tests.

In view of such problems, one object of the present invention is to enable screening of early-stage CML patients, which was difficult with blood cell counting tests.

According to a first aspect of the present invention, a specimen analysis apparatus comprising: a measurement unit configured to acquire optical information of cells contained in a specimen by irradiating the cells with a plurality of diffracted lights generated by causing light to be incident on a diffractive optical element; and
an analysis unit configured to analyze the optical information obtained by the measurement unit using an artificial intelligence algorithm to acquire first information on leukemic cells contained in the specimen.
According to a second aspect of the present invention, a specimen analysis method comprising: acquiring optical information of cells contained in a specimen by irradiating the cells with a plurality of diffracted lights generated by causing light to be incident on a diffractive optical element; and analyzing the optical information using an artificial intelligence algorithm to acquire information on leukemic cells contained in the specimen. According to a third aspect of the present invention, a method comprising: acquiring optical information of cells contained in a specimen by irradiating the cells with a plurality of diffracted lights generated by causing light to be incident on a diffractive optical element, wherein the specimen includes a first specimen collected from a patient with chronic myeloid leukemia before the start of treatment and a second specimen collected from a healthy individual; generating a classification model configured to classify leukemic cells based on the optical information obtained from the first and second specimens; acquiring an index regarding a performance of classification between leukemic cells and normal cells by the generated classification model; and outputting information regarding a therapeutic efficacy of a therapeutic drug for chronic myeloid leukemia for the patient based on the index.

According to the present invention, for example, by analyzing the optical information of cells contained in a sample using an artificial intelligence algorithm, it is possible to obtain information on leukemia cells contained in the sample.
Leukemia cells are cancerous cells found in the blood of CML patients and are genetically Philadelphia chromosome (BCR::ABL gene) positive leukocytes.
Leukemia cells cannot be morphologically distinguished from normal leukocytes, and could not be detected by blood cell counting tests or blood smear tests performed as screening tests.
Therefore, conventionally, genetic testing was performed to diagnose CML in patients who exhibited CML-characteristic findings such as leukocytosis or basophilia in blood cell counting tests.
Leukocytosis and basophilia occur as a result of abnormal proliferation of leukemia cells in the bone marrow, so by the time these findings appear, CML has often already progressed.
According to the present invention, since it is possible to obtain information on leukemia cells present in the blood of CML patients, early detection of CML can be achieved.

### BRIEF DESCRIPTION OF THE DRAWINGS

FIG. 1 is a front view schematically showing the configuration of a sample analyzer according to the first embodiment.
FIG. 2 is a block diagram showing an example of the functional configuration of a GCM measurement unit according to the first embodiment.
FIG. 3 is a block diagram showing an example of the functional configuration of a sample preparator of the GCM measurement unit according to the first embodiment.
FIG. 4 is a diagram schematically showing the configuration of an optical measurement part of the GCM measurement unit according to the first embodiment.
FIG. 5 is a diagram schematically showing the flow cell and diffracted illumination light of the GCM measurement unit according to the first embodiment.
FIG. 6 is a diagram schematically showing a distribution pattern of diffracted light included in the diffracted illumination light according to the first embodiment.
FIG. 7 is a block diagram showing an example of the functional configuration of a control unit according to the first embodiment.
FIG. 8 is a schematic diagram showing AI algorithms before and after training according to the first embodiment.
FIG. 9 is a diagram schematically showing the configuration of a cell analysis result screen according to the first embodiment.
FIG. 10 is a flowchart showing an example of control processing related to measurement by the control unit according to the first embodiment.
FIG. 11 is a diagram showing test information regarding samples in a verification experiment according to the first embodiment.
FIG. 12 is a diagram showing test information regarding samples in a verification experiment according to the first embodiment.
FIG. 13 is a diagram showing results of a verification experiment according to the first embodiment.
FIG. 14 is a diagram showing results of a verification experiment according to the first embodiment.
FIG. 15 is a diagram showing results of a verification experiment according to the first embodiment.
FIG. 16 is a front view schematically showing the configuration of a sample analyzer according to the second embodiment.
FIG. 17 is a block diagram showing an example of the functional configuration of an FCM measurement unit according to the second embodiment.
FIG. 18 is a block diagram showing an example of the functional configuration of a sample preparator of the FCM measurement unit according to the second embodiment.
FIG. 19 is a diagram schematically showing the configuration of an optical measurement part of the FCM measurement unit according to the second embodiment.
FIG. 20 is a diagram schematically showing the flow cell and direct illumination light of the FCM measurement unit according to the second embodiment.
FIG. 21 is a flowchart showing an example of control processing related to measurement by the control unit according to the second embodiment.
FIG. 22 is a flowchart showing an example of FCM measurement processing according to the second embodiment.
FIG. 23 is a diagram schematically showing the configuration of a cell analysis result screen according to the second embodiment.
FIG. 24 is a diagram schematically showing the configuration of a cell analysis result screen when GCM measurement processing is not performed according to the second embodiment.
FIG. 25 is a diagram schematically showing the configuration of a cell analysis result screen when GCM measurement processing is performed according to the second embodiment.
FIG. 26 is a front view schematically showing the configuration of a sample analyzer according to the third embodiment.
FIG. 27 is a block diagram showing an example of the functional configuration of an integrated measurement unit according to the third embodiment.
FIG. 28 is a block diagram showing an example of the functional configuration of a sample preparator of the integrated measurement unit according to the third embodiment.
FIG. 29 is a diagram schematically showing the configuration of an optical measurement part of the integrated measurement unit according to the third embodiment.
FIG. 30 is a flowchart showing an example of control processing related to measurement by the control unit according to the third embodiment.
FIG. 31 is a flowchart showing an example of control processing related to measurement by the control unit according to a modification of the third embodiment.
FIG. 32 is a block diagram showing an example of the functional configuration of a sample preparator of the integrated measurement unit according to a modification of the third embodiment.
FIG. 33 is a diagram showing a statistical comparison of PCR values in CML patient samples during the course of treatment according to the fourth embodiment.
FIG. 34 is a diagram showing an example of results of a verification experiment according to the fourth embodiment.
FIG. 35 is a flowchart showing an example of control processing related to measurement by the control unit according to the fourth embodiment.

### DETAILED DESCRIPTION

### <First Embodiment>

The first embodiment is a basic example relating to acquiring information on leukemia cells contained in a sample by irradiating cells in the sample with a plurality of diffracted lights generated by incident light on a diffractive optical element to obtain optical information of the cells, and analyzing the obtained optical information using an artificial intelligence algorithm.
In this embodiment, as an example, information on leukemia cells contained in the sample is acquired based on the technology of Ghost Cytometry.

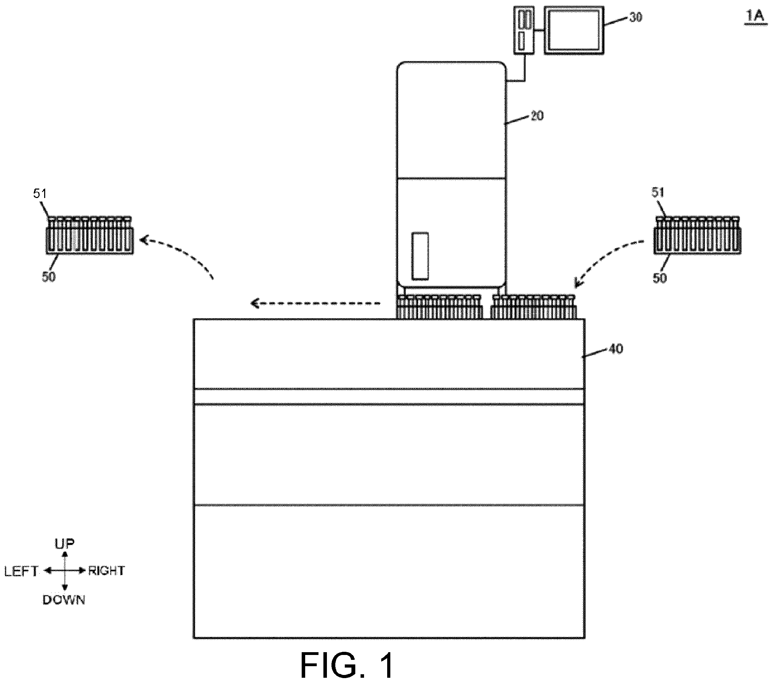

FIG. 1 is a front view schematically showing the configuration of a sample analyzer 1A as an example of the sample analyzer according to this embodiment.
The sample analyzer 1A includes, for example, a Ghost Cytometry measurement unit (hereinafter referred to as "GCM measurement unit") 20, a control unit 30, and a conveyer 40.

The sample analyzer 1A is, for example, a device that automatically analyzes samples.
The sample may be blood collected from a subject, and the sample container 51 containing the sample may be transported while being held in a sample rack 50.

An operator, such as a laboratory technician, sets the sample container 51 containing the sample into the sample rack 50 and places the sample rack 50 on the right end region of the conveyer 40.
The conveyer 40 transports the sample rack 50 and positions it in front of the GCM measurement unit 20.

The GCM measurement unit 20 takes out the sample container 51 from the sample rack 50, transfers it into the GCM measurement unit 20, and measures the sample in the sample container 51.
When the measurement of the sample in the sample container 51 is completed, the GCM measurement unit 20 returns the sample container 51 to its original position in the sample rack 50.
When all necessary measurements for all sample containers 51 on one sample rack 50 are completed, the conveyer 40 transports the sample rack 50 to the left end region of the conveyer 40. The laboratory technician removes the sample rack 50 transported to the left end region.

The GCM measurement unit 20 is configured to be able to measure samples transported on the conveyer 40.
The conveyer 40 is configured to automatically supply the sample rack 50 containing the sample to the GCM measurement unit 20.
By automatically supplying the sample to the GCM measurement unit 20 via the conveyer 40, the labor required by the laboratory technician to transfer the sample to the GCM measurement unit 20 can be reduced.

The control unit 30 controls, for example, the GCM measurement unit 20 and the conveyer 40.
The control unit 30 also analyzes measurement information obtained by the GCM measurement unit 20.

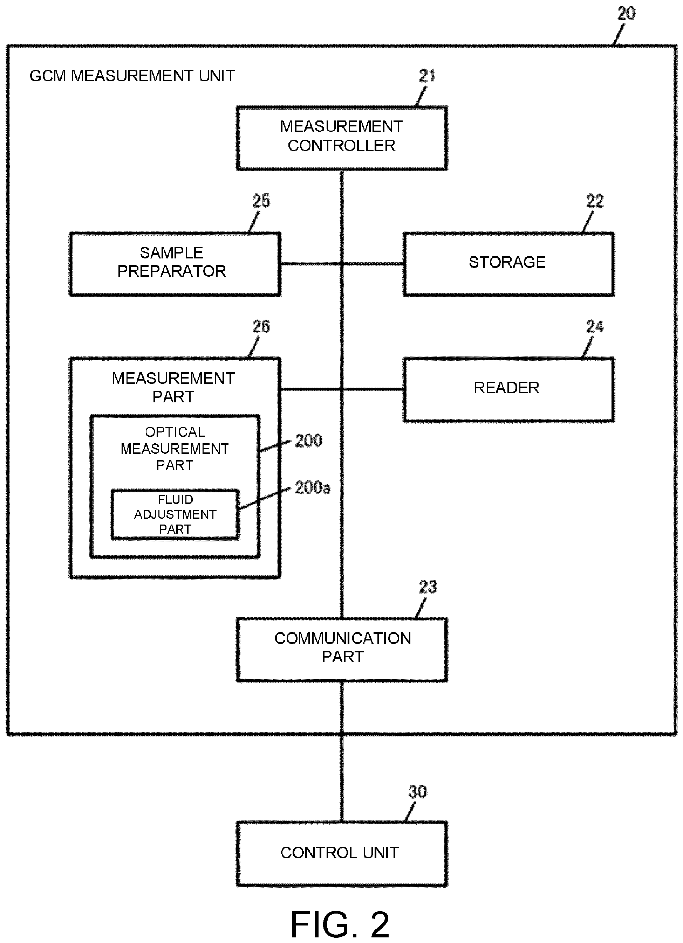

FIG. 2 is a block diagram showing an example of the functional configuration of the GCM measurement unit 20.

The GCM measurement unit 20 includes, for example, a measurement controller 21, storage 22, communication part 23, reader 24, sample preparator 25, and measurement part 26.

The measurement controller 21 includes, for example, an FPGA or CPU. The storage 22 includes, for example, an HDD, SSD, RAM, or ROM.
The measurement controller 21 performs various processes based on a program stored in the storage 22 and controls each part of the GCM measurement unit 20.

The communication part 23 includes, for example, a connection terminal based on the USB standard, and communicates with the control unit 30.

The reader 24 includes, for example, a barcode reader, reads a barcode from a barcode label attached to the sample container 51, and acquires a sample ID.

The sample preparator 25 aspirates a sample from the sample container 51 and mixes a reagent with the aspirated sample to prepare a measurement sample.

The measurement part 26 includes, for example, an optical measurement part 200, and the optical measurement part 200 includes, for example, a fluid adjustment part 220a.

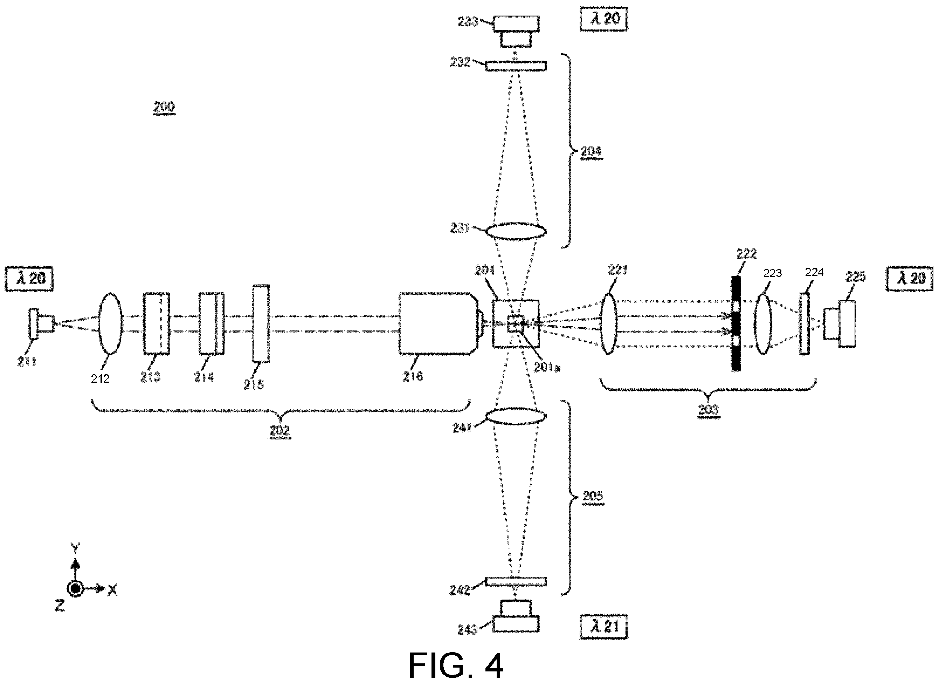

The fluid adjustment part 200a includes, for example, a container for containing sheath liquid, a syringe for transferring the measurement sample, and a pneumatic source (pump) for transferring the sheath liquid.
The fluid adjustment part 200a supplies the sheath liquid together with the measurement sample prepared by the sample preparator 25 to the flow cell 201 (see FIG. 4) of the optical measurement part 200, and adjusts the flow rate of the measurement sample flowing through the flow cell 201 per unit time.
The optical measurement part 200 measures the measurement sample supplied to the flow cell 201.

The optical measurement part 200 includes an amplifier and an A/D converter, performs signal processing on the detection signal obtained by measurement, and outputs the processed measurement information to the measurement controller 21.
The measurement information is, for example, a Ghost Motion Imaging (GMI) waveform signal based on Ghost Cytometry (GCM).
This may be referred to as GMI waveform information, and is an example of optical information of cells obtained by irradiating cells contained in a sample with a plurality of diffracted lights generated by incident light on a diffractive optical element. Hereinafter, it will be simply referred to as a "waveform signal."

The measurement controller 21 stores the waveform signal output from the measurement part 26 in the storage 22.
When the measurement of one sample is completed, the measurement controller 21 transmits the waveform signal stored in the storage 22 to the control unit 30 in association with the sample ID read by the reader 24.

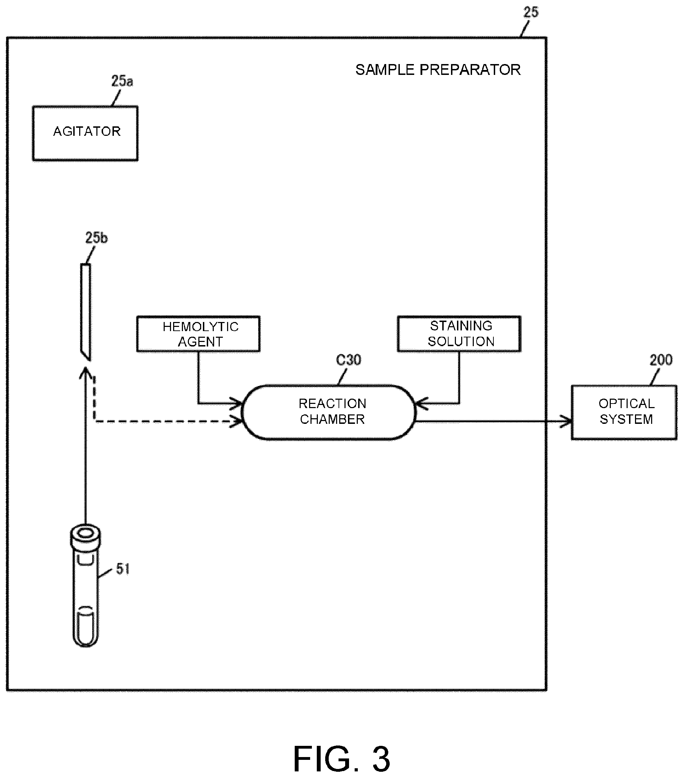

FIG. 3 is a block diagram showing an example of the functional configuration of the sample preparator 25 for preparing a measurement sample.

The sample preparator 25 includes, for example, an agitator 25a, an aspiration pipette 25b, and a reaction chamber C30.

The agitator 25a is configured to grip the sample container 51 and agitate the sample in the gripped sample container 51 by oscillating it.
The aspiration pipette 25b is, for example, a nozzle with a pointed lower end, and is configured to penetrate the lid of the sample container 51 made of an elastic material. The aspiration pipette 25b aspirates the sample from the sample container 51 after agitation and dispenses the aspirated sample into the reaction chamber C30.

In the reaction chamber C30, the sample, a hemolytic agent (an example of a reagent) for hemolyzing red blood cells, and a staining solution (staining agent: an example of a reagent) containing a fluorescent dye for staining a predetermined part of the cell are mixed to prepare a measurement sample.
The hemolytic agent mixed in the reaction chamber C30 is, for example, a WDF hemolytic agent.
The staining solution mixed in the reaction chamber C30 is, for example, a WDF staining solution.
The measurement sample prepared in the reaction chamber C30 is measured by the optical measurement part 200.

The optical measurement part 200 acquires a detection signal corresponding to blood cells in the measurement sample, performs signal processing on the acquired detection signal, and obtains a waveform signal.
The controller 31 and arithmetic unit 32 of the control unit 30 analyze the waveform signal obtained by measuring this measurement sample, classify whether it is a CML cell or not, and obtain the number of each blood cell.

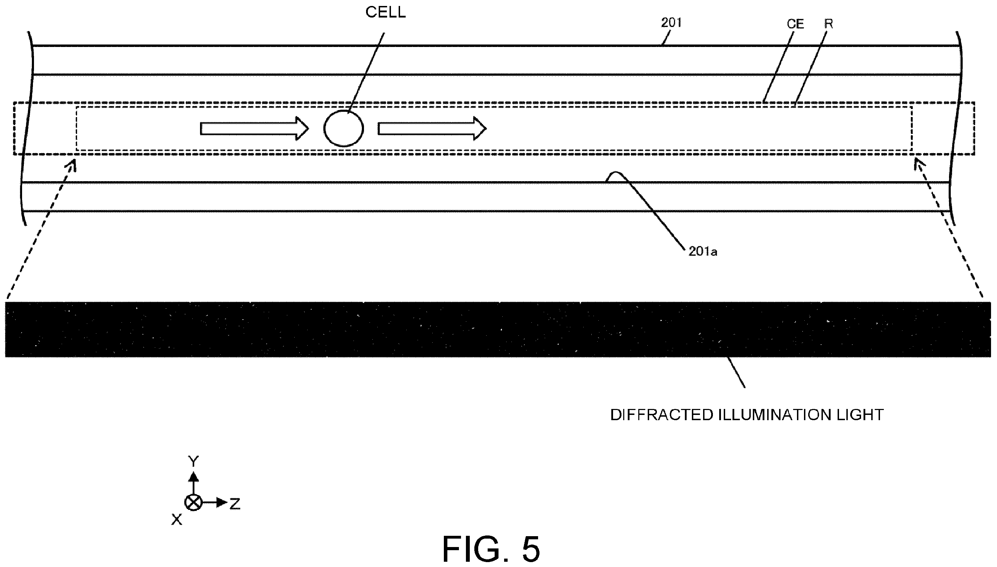

In this case, the waveform signal includes, for example, time-series data of forward scattered light corresponding to each cell, indicating the intensity change of forward scattered light received by the light detector 225 while each cell in the measurement sample flowing through the flow cell 201 (see FIGS. 4 and 5) passes through the illumination range R of the illumination light; time-series data of side scattered light corresponding to each cell, indicating the intensity change of side scattered light received by the light detector 233 while each cell in the measurement sample flowing through the flow cell 201 passes through the illumination range R; and time-series data of fluorescence corresponding to each cell, indicating the intensity change of fluorescence received by the light detector 243 while each cell in the measurement sample flowing through the flow cell 201 passes through the illumination range R.

As described later, the controller 31 and arithmetic unit 32 of the control unit 30 input, for example, the time-series data of forward scattered light and side scattered light to the trained AI algorithm 62 for analysis.

Note that the waveform signal may be any information reflecting the size, shape, internal structure, or nucleic acid content of each cell, obtained by irradiating each cell in the measurement sample with light in which a plurality of diffracted lights generated by the diffractive optical element 215 to which light is incident are distributed, and is not limited to the above time-series data.

Further, the hemolytic agent and staining solution mixed in the reaction chamber C30 are not limited to the above reagents.

In addition, the mixing of the staining solution in the reaction chamber C30 may be omitted. That is, the reagent may not contain a staining agent, and the sample and reagent (hemolytic agent) may be mixed in the reaction chamber C30 to prepare the measurement sample.
Alternatively, a diluent may be mixed instead of the staining solution. That is, the sample and reagent (hemolytic agent, diluent) may be mixed in the reaction chamber C30 to prepare the measurement sample.
In these cases, information on leukemia cells contained in the sample can be obtained without labeling (no need for fluorescent labeling), and the fluorescence collecting optical system 205 and light detector 243 described later in FIG. 4 can be omitted.

FIG. 4 is a diagram schematically showing the configuration of the optical measurement part 200. For convenience, mutually orthogonal X, Y, and Z axes are shown in FIG. 4. The Z-axis direction is the flow direction of the measurement sample in the flow cell 201.

The optical measurement part 200 includes, for example, a flow cell 201, a light source 211, an illumination optical system 202, a forward condenser optical system 203, a side condenser optical system 204, a fluorescence condenser optical system 205, and light detectors 225, 233, and 243.

The illumination optical system 202 includes, for example, a collimator lens 212, cylindrical lenses 213 and 214, a diffractive optical element (DOE) 215, and a condenser lens 216. The illumination optical system 202 irradiates the flow path 201a of the flow cell 201 with light from the light source 211.
Hereinafter, the light emitted from the light source 211 and irradiated onto the flow path 201a is referred to as "diffracted illumination light." The diffracted illumination light is light in which a plurality of diffracted lights generated by the diffractive optical element 215 are distributed. More specifically, the diffracted illumination light is light having a structured illumination pattern (structured light illumination).

The forward condenser optical system 203 includes, for example, a condenser lens 221, a beam stopper 222, a condenser lens 223, and an optical filter 224. The forward condenser optical system 203 condenses forward scattered light generated from blood cells onto the light detector 225 and blocks the diffracted illumination light that has passed through the flow cell 201 without being irradiated onto the blood cells.

The side condenser optical system 204 includes, for example, a condenser lens 231 and an optical filter 232. The side condenser optical system 204 condenses side scattered light generated from blood cells onto the light detector 233.

The fluorescence condenser optical system 205 includes, for example, a condenser lens 241 and an optical filter 242. The fluorescence condenser optical system 205 condenses fluorescence generated from blood cells onto the light detector 243.

The light source 211 is, for example, a semiconductor laser light source. The light source 211 emits light of a predetermined wavelength λ20 in the X-axis direction. The wavelength λ20 is, for example, 405 nm. The fast axis direction and slow axis direction of the light source 211 are parallel to the Y-axis and Z-axis directions, respectively. The collimator lens 212 converts the light emitted from the light source 211 into parallel light.

The cylindrical lens 213 is a concave cylindrical lens, and the cylindrical lens 214 is a convex cylindrical lens. The cylindrical lens 213 increases the width of the light emitted from the light source 211 in the Z-axis direction without changing the width in the Y-axis direction, forms it into a substantially circular shape, and causes it to enter the cylindrical lens 214. The cylindrical lens 214 converts the light emitted from the light source 211 into parallel light.

The collimator lens 212 and cylindrical lenses 213 and 214 are arranged so that the light emitted from the light source 211 and transmitted through the collimator lens 212 and cylindrical lenses 213 and 214 has a substantially circular shape when viewed in the X-axis direction. As a result, the light incident on the diffractive optical element 215 has a substantially circular shape.

Note that the configuration of the light source 211, collimator lens 212, and cylindrical lenses 213 and 214 may be any configuration in which the light incident on the diffractive optical element 215 has a substantially circular shape, and other configurations may be used. For example, each of the light source 211, collimator lens 212, and cylindrical lenses 213 and 214 may be rotated 90 degrees about the X-axis direction. In this case, the fast axis direction and slow axis direction of the light source 211 are parallel to the Z-axis and Y-axis directions, respectively. Alternatively, a light source that emits light in a substantially circular shape may be used as the light source 211, and the collimator lens 212 and cylindrical lenses 213 and 214 may be omitted.

The diffractive optical element 215 is formed with a complex uneven diffractive pattern, such as grooves or inclines, for imparting a diffraction effect to the incident light. The diffractive optical element 215 can be manufactured, for example, based on the disclosure of U.S. Patent No. 9,477,018. The disclosure of U.S. Patent No. 9,477,018 is incorporated herein by reference.
The diffractive optical element 215 diffracts the light in the X-axis direction incident from the cylindrical lens 214 side in the X-axis direction to generate a plurality of diffracted lights having different propagation directions.
The plurality of diffracted lights are obtained by dispersing the incident light, and the diffraction orders of the plurality of diffracted lights are different from each other.
The focusing lens 216 focuses the plurality of diffracted lights generated from the diffractive optical element 215 onto the flow cell 201.
The plurality of diffracted lights generated by the diffractive optical element 215, each having a different propagation direction, are focused onto the flow cell 201 to form the diffracted illumination light.

The measurement sample prepared in the reaction chamber C30 of FIG. 3 flows through the flow cell 201.
The cells in the measurement sample flowing through the flow cell 201 are irradiated with the diffracted illumination light, and forward scattered light, side scattered light, and fluorescence are generated from the portions of the cells irradiated with each of the diffracted lights in the diffracted illumination light.
The forward scattered light is generated in the X-axis direction, and the side scattered light and fluorescence are generated in a direction intersecting the X-axis direction (for example, the Y-axis direction).

The focusing lens 221 converges the forward scattered light generated from the cells and the diffracted illumination light that has passed through the flow cell 201 without being irradiated onto the cells.
The beam stopper 222 allows the forward scattered light generated from the cells to pass through and blocks the diffracted illumination light that has passed through the flow cell 201.
The condenser lens 223 condenses the forward scattered light that has passed through the beam stopper 222 onto the light detector 225.
The optical filter 224 is configured to transmit only light of wavelength λ20.
The light detector 225 receives the forward scattered light transmitted through the optical filter 224 and outputs a detection signal according to the received intensity. The light detector 225 is, for example, a photomultiplier tube (PMT).

The condenser lens 231 condenses the side scattered light generated from the cells onto the light detector 233.
The optical filter 232 is configured to transmit only light of wavelength λ20.
The light detector 233 receives the side scattered light transmitted through the optical filter 232 and outputs a detection signal according to the received intensity. The light detector 233 is, for example, a photomultiplier tube (PMT).

The condenser lens 241 condenses the fluorescence generated from the cells onto the light detector 243.
The optical filter 242 is configured to transmit only light of wavelength λ21.
The light detector 243 receives the fluorescence transmitted through the optical filter 242 and outputs a detection signal according to the received intensity. The light detector 243 is, for example, a photomultiplier tube (PMT).

Note that the light detectors 225, 233, and 243 are not limited to photomultiplier tubes (PMTs), and may be, for example, photodiodes (PDs).

FIG. 5 is a diagram schematically showing the flow cell 201 and diffracted illumination light. The X, Y, and Z axes, as in FIG. 4, are indicated in FIG. 5.

Inside the flow cell 201, a flow path 201a through which the measurement sample flows is formed parallel to the Z-axis. By flowing sheath liquid together with the measurement sample in the flow path 201a, the cells contained in the measurement sample are enveloped by the sheath liquid and pass through the central region CE of the flow path 201a.
The diffracted illumination light condensed by the condensing lens 216 is irradiated onto a predetermined irradiation region R located in the central region CE of the flow path 201a.
The flow rate of the measurement sample per unit time is adjusted so that only one cell is positioned in the irradiation region R at a time, in other words, so that two or more cells do not pass through the irradiation region R simultaneously.

The lower part of FIG. 5 shows an example of an image obtained by irradiating the diffracted illumination light generated by the diffractive optical element 215 in a dark room and capturing it with a camera.
In the image of the diffracted illumination light in FIG. 5, the black areas indicate regions where no light is present, and the white dots indicate regions where light is present. The white dots in the image of the diffracted illumination light indicate the diffracted lights generated by the diffractive optical element 215.
In the example shown in FIG. 5, the diffracted lights include the 0th order, +1 to +300th order, and -1 to -300th order diffracted lights, and are shown as a total of 601 white dots in the image of the diffracted illumination light.
The diffractive optical element 215 is formed with a diffraction pattern (steps or grooves) so that the diffracted lights are distributed as shown in such an image of the diffracted illumination light.

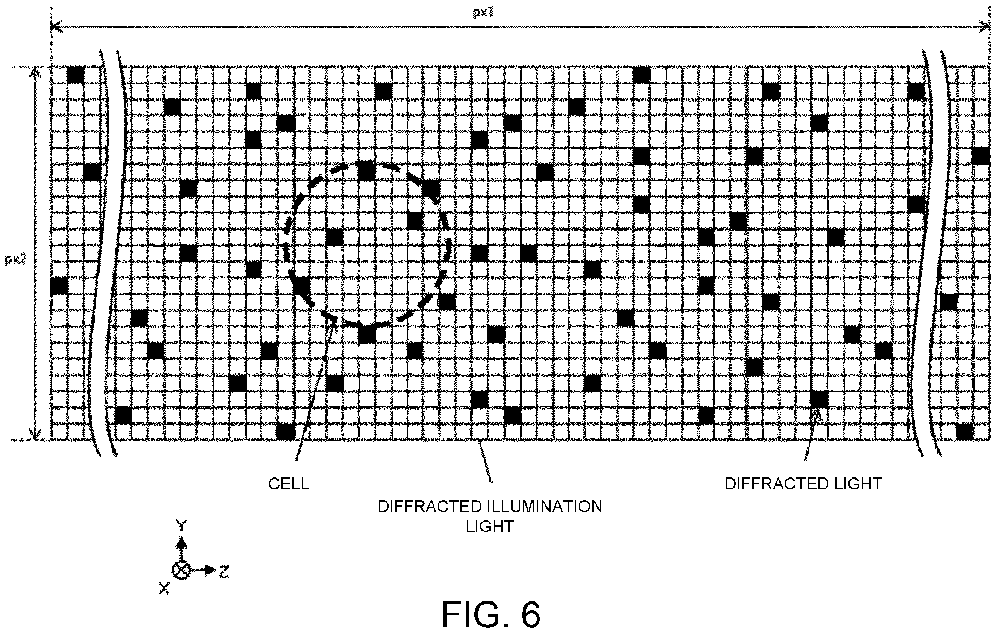

FIG. 6 is a diagram schematically showing the distribution pattern of diffracted lights included in the diffracted illumination light.

FIG. 6 represents an image in which the irradiation region R (see FIG. 5) is divided into a grid by a plurality of squares having sides of the same length as the diameter of the diffracted light spots.
The black squares indicate regions containing diffracted light spots. The white squares indicate regions not containing diffracted light spots.

In FIG. 6, a cell passing through the irradiation region R is shown as a dashed circle. Since the diameter of the diffracted light spots in the image of the diffracted illumination light illustrated in FIG. 6 is about 1 µm, the size of each square is 1 µm × 1 µm in this case. The size of the cell is about 10 µm.
The size of the diffracted illumination light in the irradiation region R and the length in the Y-axis or Z-axis direction can be expressed as the number of pixels when each grid region is regarded as one pixel.
In the example shown in FIG. 6, the length of the diffracted illumination light in the flow direction (Z-axis direction) of the measurement sample is px1 (pixels), the length in the short direction (Y-axis direction) is px2 (pixels), and the size of the diffracted illumination light is px1 × px2 (pixels).

The diffractive optical element 215 is designed so that the plurality of diffracted lights constituting the diffracted illumination light are distributed in a predetermined pattern. Here, the predetermined pattern is a random pattern.
The pattern may have no repetition of a specific pattern at all, or may have periodic repetition of a specific pattern. However, it is preferable that at least one diffracted light is arranged in a region extending in the Z-axis direction with a length of one pixel in the Y-axis direction so that the entire region of the cell is exposed to the diffracted illumination light at least once.

When the measurement sample flows through the flow path 201a of the flow cell 201 during measurement, the cells in the measurement sample move in the Z-axis direction through the irradiation region R of the diffracted illumination light.
At this time, the fluid adjustment part 200a (see FIG. 2) adjusts the flow rate per unit time to be substantially constant.
When the cells flowing in the Z-axis direction are irradiated with the diffracted lights included in the diffracted illumination light, forward scattered light and side scattered light are generated from the portions of the cells irradiated with the diffracted lights.
In addition, when the diffracted lights are irradiated onto the fluorescent dye-stained cells, fluorescence is generated from the fluorescent dye irradiated with the diffracted lights. The light detector 225 (see FIG. 4) receives the forward scattered light generated by the plurality of diffracted lights irradiated onto the cell, the light detector 233 (see FIG. 4) receives the side scattered light generated by the plurality of diffracted lights irradiated onto the position of the cell, and the light detector 243 (see FIG. 4) receives the fluorescence generated by the plurality of diffracted lights irradiated onto the predetermined position of the stained cell.

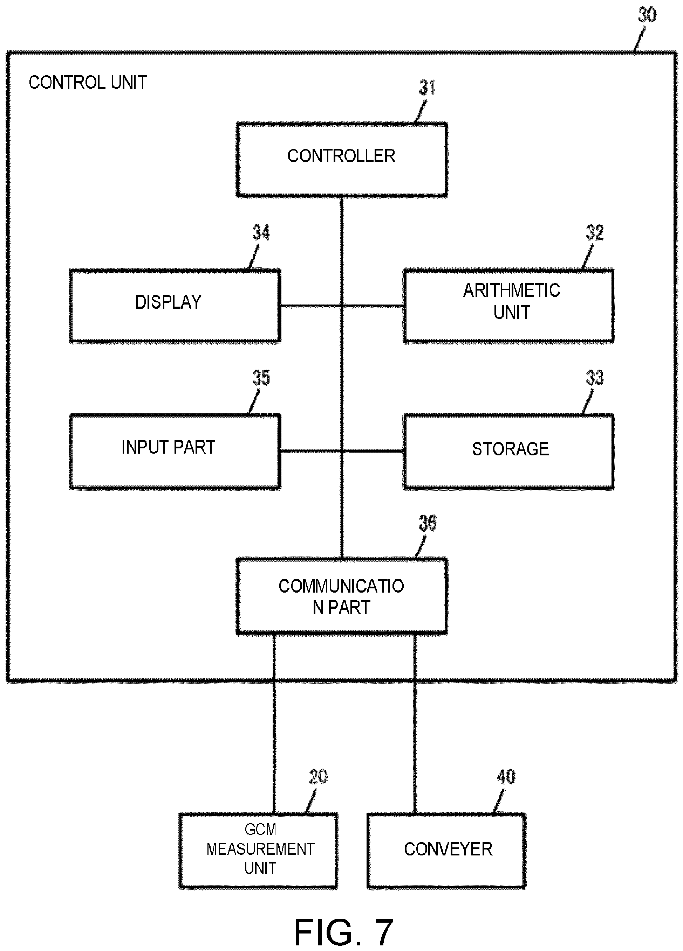

As the cells flow in the Z-axis direction, the number of diffracted lights irradiated onto the cells changes, and the positions of the cells irradiated by each diffracted light also change, so the intensity of the forward scattered light, side scattered light, and fluorescence generated from the cells changes over time. Therefore, the detection signals of the light detectors 225, 233, and 243 also change over time.
As described later, the arithmetic unit 32 (see FIG. 7) classifies the cells using the AI algorithm 62 based on the waveform signals obtained from these detection signals.

FIG. 7 is a block diagram showing an example of the functional configuration of the control unit 30.

The control unit 30 includes, for example, a controller 31, an arithmetic unit 32, storage 33, display 34, input part 35, and communication part 36.

The controller 31 includes, for example, a CPU.
The arithmetic unit 32 includes, for example, a GPU (Graphics Processing Unit) or NPU (Neural network Processing Unit).
The torage 33 includes, for example, an HDD, SSD, RAM, or ROM.
The controller 31 executes a program stored in the storage 33 to control each part of the control unit 30 and analyzes cells based on the measurement information obtained by the GCM measurement unit 20.

The controller 31 causes the arithmetic unit 32 to perform analysis using the AI algorithm 62, analyzes the waveform signals obtained by the optical measurement part 200 of the GCM measurement unit 20, and obtains the GCM analysis result.
The AI algorithm 62 in this case may include a statistical machine learning model such as SVM (Support Vector Machine) or a neural network model such as MLP (Multi-Layer Perceptron).

Note that the controller 31 and the arithmetic unit 32 may be integrated as a control arithmetic unit, and this control arithmetic unit may have the functions of both the controller 31 and the arithmetic unit 32.

The display 34 includes, for example, a liquid crystal display.
The Input part 35 includes, for example, a pointing device including a keyboard, mouse, and touch panel. Note that the liquid crystal display of the display 34 and the touch panel of the input part 35 may be integrally formed.
The communication part 36 includes, for example, a connection terminal based on the USB standard, and communicates with the GCM measurement unit 20 and the conveyer 40.

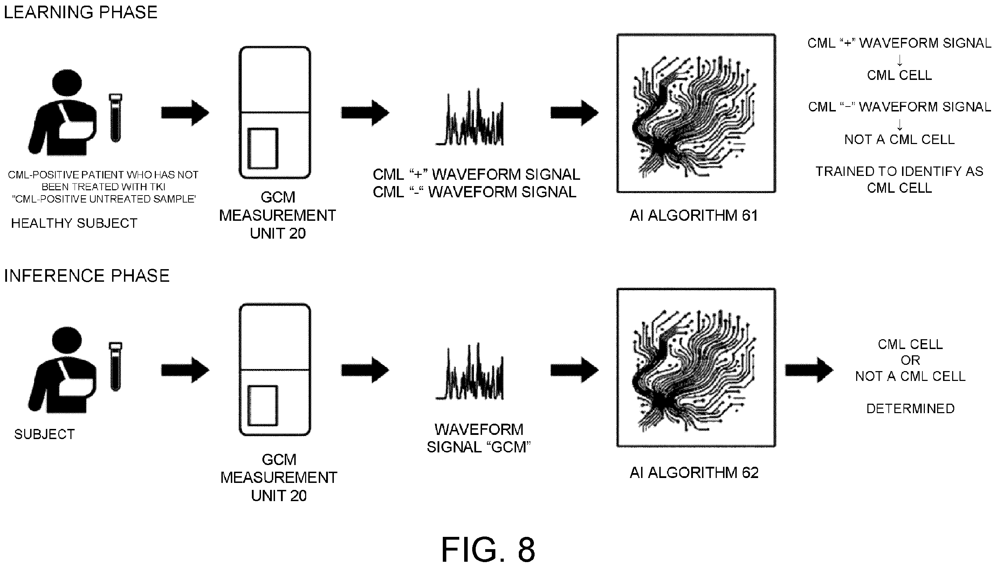

FIG. 8 is a schematic diagram showing the AI algorithm 61 before training and the AI algorithm 62 after training.

In training the AI algorithm 61, a sample in which most of the leukocytes in peripheral blood are considered to have been replaced by CML cells based on genetic testing can be used for training the AI algorithm.
For example, a sample in which the proportion of BCR::ABL fusion gene-positive leukocytes among all leukocytes is equal to or greater than a predetermined value can be used.
For example, a sample in which Major BCR::ABL1 mRNA (%) is 80% or more by PCR test can be used, and more preferably 90% or more.
As such a sample, peripheral blood collected from a CML-positive patient who has not been treated with TKI (hereinafter referred to as "CML-positive untreated sample") is preferably used.

The controller 31, for example, causes the conveyer 40 to transport the sample rack 50 holding the sample container 51 with the CML-positive untreated sample, and supplies the sample to the GCM measurement unit 20. The controller 31 then acquires, for example, a training waveform signal indicating CML positive (hereinafter, for convenience, referred to as "CML (+) waveform signal") from the GCM measurement unit 20.

The controller 31 also causes, for example, the conveyer 40 to transport the sample rack 50 holding the sample container 51 collected from a healthy subject, and supplies the sample to the GCM measurement unit 20. The controller 31 then acquires, for example, a training waveform signal indicating CML negative (hereinafter, for convenience, referred to as "CML (-) waveform signal") from the GCM measurement unit 20. The sample collected from a healthy subject can be regarded as containing normal leukocytes (and not containing CML cells).
For example, the "CML (+) waveform signal" and "CML (-) waveform signal" may be a set of waveform signals (training data set) including waveform signals of a plurality of cells.

As shown in the upper part of FIG. 8, the training waveform signals ("CML (+) waveform signal" and "CML (-) waveform signal") used to train the AI algorithm 61 before training are, for example, information obtained by measuring specific cells (normal leukocytes and CML cells) with the GCM measurement unit 20.

Note that, for example, among the "CML (+) waveform signal" and "CML (-) waveform signal" of whole blood, only the waveform signals corresponding to leukocytes (e.g., all leukocytes, granulocytes, lymphocytes, monocytes) may be used as the "CML (+) waveform signal" or "CML (-) waveform signal".

In this case, for example, by using the waveform signal (GMI waveform signal), it is possible to determine whether the cell to be determined is a leukocyte and, if so, which type of leukocyte it is.
The types of leukocytes may be, for example, based on leukocyte classifications, granulocytes (neutrophils, eosinophils, basophils), lymphocytes, and monocytes.
As a method for such determination, for example, the method disclosed in "Pooled CRISPR screening of high-content cellular phenotypes using ghost cytometry" Tsubouchi A, An Y, Kawamura Y [..] Ota S. Cell Rep Methods 2024-03-25 (https://doi.org/10.1016/j.crmeth.2024.100737) may be applied.

The AI algorithm 61 is, for example, a neural network including multiple intermediate layers. The neural network in this case may be, for example, a convolutional neural network (CNN) having a convolutional layer.
The AI algorithm 61 has an input layer, an output layer, and an intermediate layer.
A data set of waveform signals (such as "CML (+) waveform signal" and "CML (-) waveform signal") obtained by sampling the analog detection signal from each of cells at a predetermined sampling period is input to the input layer, and label values corresponding to the cell types (normal leukocyte or CML cell) are input to the output layer, thereby training the AI algorithm 61.
By repeatedly performing such training in advance, the trained AI algorithm 62 is generated.

As shown in the lower part of FIG. 8, for example, the trained AI algorithm 62 also has an input layer, an output layer, and an intermediate layer.
The waveform signal obtained based on the subject's sample is input to the input layer. As a result, the output layer outputs classification information regarding the type of cell corresponding to the waveform signal (whether it is a CML cell or not).

For example, when the output layer has one node, the label value corresponding to a CML cell may be "1" and the label value corresponding to a normal leukocyte may be "0".
Also, for example, when the output layer has two nodes, the AI algorithm may be trained so that when a "CML (+) waveform signal" is input to the input layer, the node corresponding to CML cells outputs "1" and the node corresponding to normal leukocytes outputs "0". Similarly, when a "CML (-) waveform signal" is input to the input layer, the node corresponding to CML cells outputs "0" and the node corresponding to normal leukocytes outputs "1".

The classification information may include the probability that the target cell is a CML cell. For example, when the output layer has one node, the classification information is the output value of the output layer and takes a value in the range of "0 to 1". When the output layer has two nodes, the classification information is the output value of the node corresponding to CML cells and takes a value in the range of "0 to 1". The closer the classification information is to "1", the higher the probability that the cell is a CML cell.
Furthermore, based on the classification information, the controller 31 may perform, for example, a threshold determination to determine whether the target cell is a CML cell in the waveform signal corresponding to a cell in the subject's sample.

The training of such an AI algorithm 61 and classification using the AI algorithm 62 are performed, for example, by inputting, as input data to the input layer, a set of waveform signal data obtained for each cell by one or more of the three light detectors 225, 233, and 243 (see FIG. 4).
Specifically, when using the waveform signal obtained from any one of the light detectors 225, 233, or 243, and obtaining n data sets from the detection signal for each cell, the number of data of the detection signal input to the AI algorithm 61 or 62 for one cell is n, and the number of nodes in the input layer is also n.
For example, when the data sets of the three detection signals obtained from each of the three light detectors 225, 233, and 243 are input as input data to the input layer, 3n data sets are obtained from the three detection signals, and the number of nodes in the input layer is also 3n.

In this embodiment, the arithmetic unit 32 performs cell classification using the AI algorithm 62, but the controller 31 may perform cell classification using the AI algorithm 62. However, the arithmetic unit 32, which comprises a GPU or the like, can classify cells using the AI algorithm 62 more quickly. The above-mentioned control arithmetic unit may also perform cell classification using the AI algorithm 62.

The AI algorithm 62 is not limited to a neural network model. For example, it may be a machine learning model (classification model) such as SVM. In this case, in the learning phase, the "CML (+) waveform signal" and "CML (-) waveform signal" may be used as explanatory variables, and the objective variable may be set so that the two can be classified.

The control unit 30 repeatedly determines, for each waveform signal corresponding to a cell in the subject's sample, whether the target cell is a CML cell, and accumulates the results to calculate the proportion (e.g., "%") of blood cells that are CML cells in the sample.

The waveform signal (GMI waveform signal) may be a waveform signal corresponding to each cell in whole blood. That is, it is possible to determine whether each cell is a CML cell regardless of whether it is a leukocyte, and to calculate the number of CML cells among all cells or the proportion of CML cells to the total number of cells. The waveform signal may also be a waveform signal corresponding to cells determined to be leukocytes. That is, it is possible to determine whether only the cells determined to be leukocytes are CML cells, and to calculate the number of CML cells among all leukocytes or the proportion of CML cells to the total number of leukocytes.
The waveform signal may also be a waveform signal corresponding to a specific type of leukocyte (granulocyte, lymphocyte, monocyte). That is, it is possible to determine whether only the cells determined to be a specific type are CML cells, and to calculate the number of CML cells among a specific type of cell or the proportion of CML cells to the number of a specific type of cell.

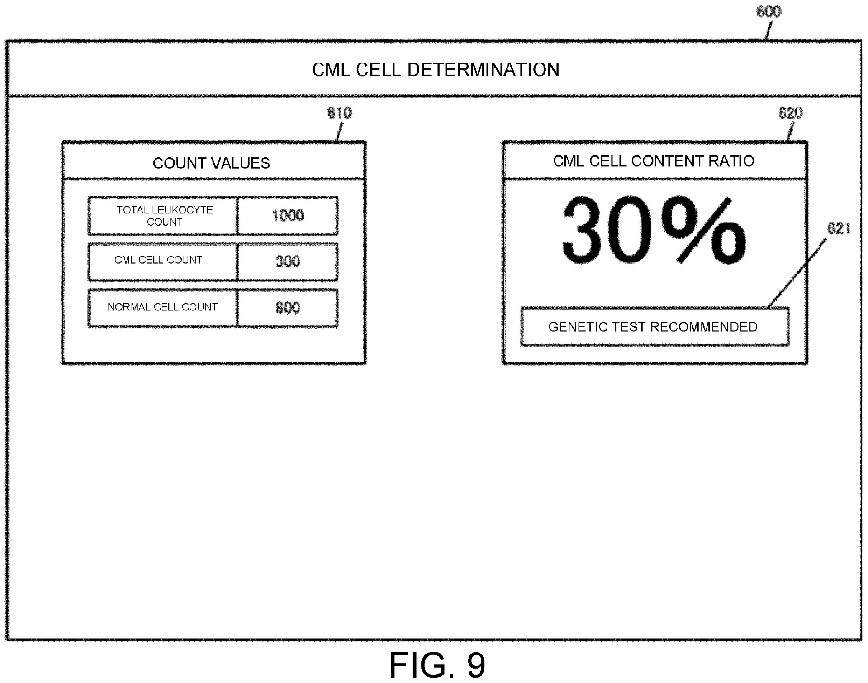

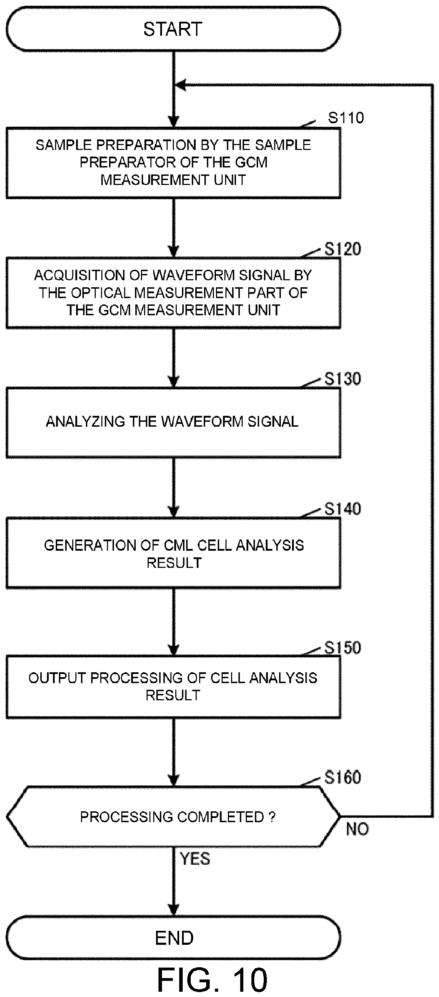

FIG. 9 is a diagram schematically showing the configuration of a cell analysis result screen 600 that is displayed on the display 34 in the analysis result output processing (step S150 in FIG. 10) described later. The cell analysis result screen 600 may be referred to as an output screen for CML cell analysis result information based on classification information.

The cell analysis result screen 600 includes, for example, a count value display region 610 and a CML cell content ratio display region 620.
In this example, the count value display region 610 is configured to display, for example, the number of leukocytes (total leukocyte count) analyzed in the sample, the number of cells determined to be CML cells among the total leukocyte count (CML cell count), and the number of cells determined not to be CML cells (normal cell count) among the total leukocyte count.

The CML cell content ratio display region 620 is configured to display, for example, the ratio of CML cells contained in the sample (CML cell content ratio) in units of "%", based on the CML cell count and the total leukocyte count.

In the CML cell content ratio display region 620, when the CML cell content ratio exceeds a predetermined ratio (for example, "20%") or is equal to or greater than the predetermined ratio, flag information 621 may be displayed to recommend a more sensitive genetic test, such as Major BCR::ABL1 mRNA quantitative PCR or FISH method, compared to this method, for the subject from whom the sample was collected.

Next, with reference to FIG. 10, the measurement processing of the sample analyzer 1A will be described.
FIG. 10 is a flowchart showing an example of the flow of control processing related to measurement by the control unit 30.

First, in step S110, the controller 31 of the control unit 30 controls the GCM measurement unit 20 to cause the sample preparator 25 (see FIGS. 2 and 3) to prepare the sample. As a result, the measurement sample is prepared in the reaction chamber C30.

In step S120, the controller 31 controls the GCM measurement unit 20 so that measurement is performed by the optical measurement part 200. As a result, as shown in FIG. 5, the optical measurement part 200 irradiates the cells in the sample contained in the measurement sample flowing through the flow cell 201 with diffracted illumination light and acquires the waveform signal. The controller 31 then acquires the waveform signal obtained by the measurement part 26 from the GCM measurement unit 20.

In step S130, the controller 31 and arithmetic unit 32 input the waveform signal acquired in step S120 to the trained AI algorithm 62 for analysis.

In step S140, the controller 31 and arithmetic unit 32 generate CML cell analysis information based on the analysis in step S130.
The CML cell analysis information may include the count values of blood cells, CML cells, and non-CML cells, and/or the CML cell content ratio, obtained by analyzing the waveform signal based on the measurement by the optical measurement part 200. The CML cell analysis information may be information based on classification information, and may be referred to as GML analysis information.

For example, when a laboratory technician inputs a display instruction via the input part 35, in step S150, the controller 31 displays (as an example of output) the cell analysis result screen 300 including the CML cell analysis information generated in step S140 on the display 34.

Note that the "output" of information including analysis results and the like may include, in addition to display (display output) on the device itself, output of information to other functional parts of the device (internal output), output (external output) or transmission (external transmission) of information to devices other than the device (external devices), and the like. Audio output (including voice output) may also be included.

In step S160, for example, based on an input operation by the laboratory technician via the input part 35, the controller 31 determines whether to end the process. If it is determined to continue the process (S160: NO), the controller 31 controls, for example, the GCM measurement unit 20 to take out another sample container 51 from the sample rack 50 and transfer it into the GCM measurement unit 20, for preparing to measure the sample in the new sample container 51. Then, for example, the process returns to step S110.
If it is determined to end the process (S160: YES), the controller 31 controls, for example, the GCM measurement unit 20 to return the sample container 51 from the GCM measurement unit 20 to its original position in the sample rack 50, and ends the process.

### <Verification Experiment>

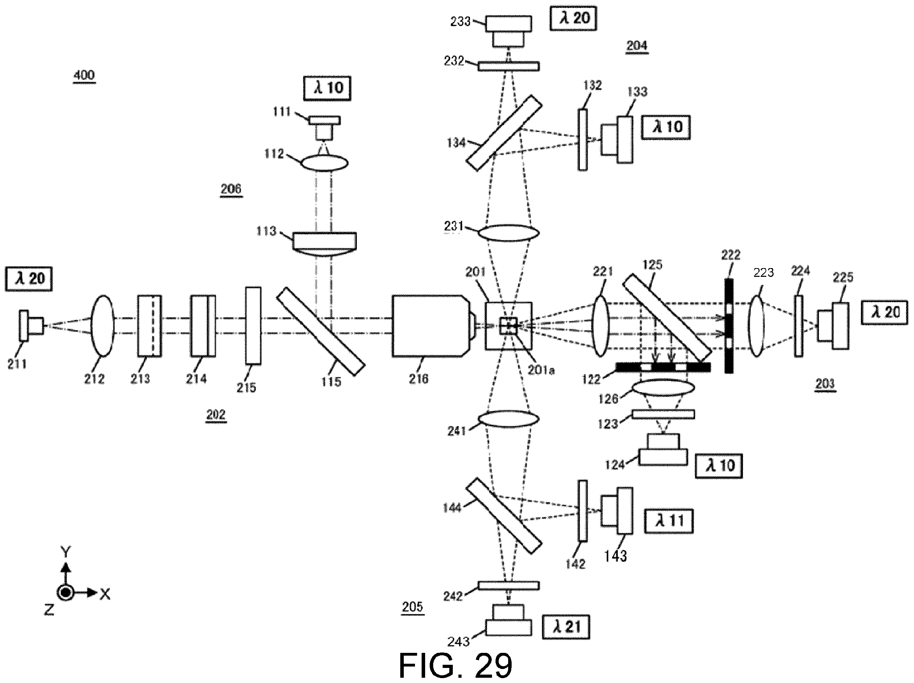

To confirm the effectiveness of this method, a verification experiment was conducted by assuming CML patients undergoing TKI therapy, which is used as standard treatment for CML, as early-stage CML patients, and examining whether samples from CML patients under TKI therapy and samples from healthy subjects can be distinguished.
In the verification experiment, as will be described later with reference to FIG. 29, an optical measurement part 400 capable of acquiring flow cytometry information in addition to GMI waveform information from a single cell was used.

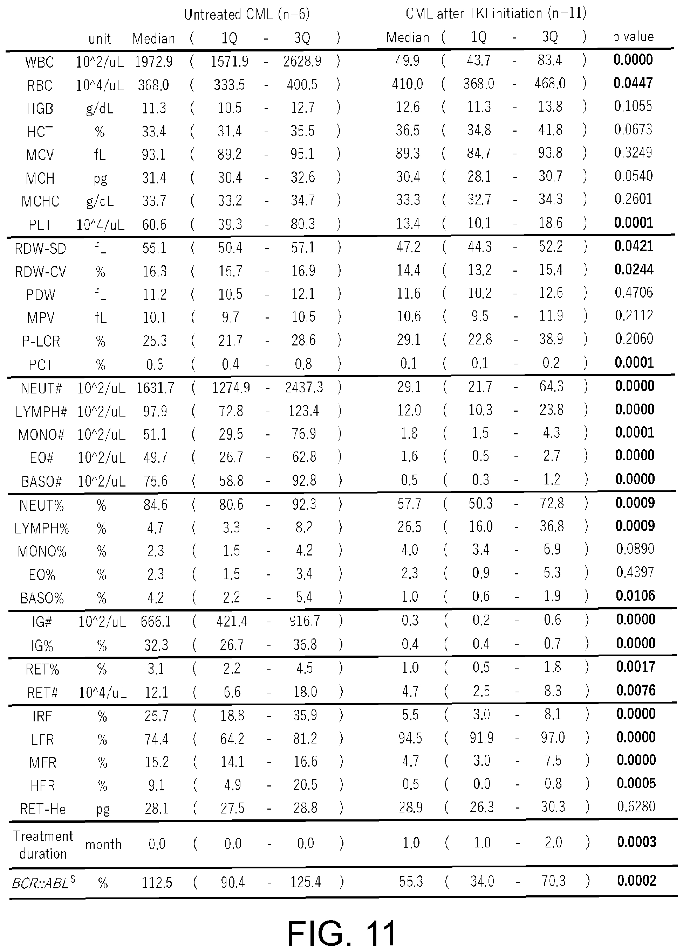

FIG. 11 shows a comparison of blood test values between samples from CML patients before TKI therapy (n=6) and samples from CML patients under TKI therapy (n=11). The bottom row shows the values from genetic testing by PCR.

In CML patients before TKI therapy, the proportion of CML cells is 90% or more in BCR::ABL1 mRNA (IS%), indicating that almost all leukocytes in the blood of CML patients are CML cells.

On the other hand, in CML patients under TKI therapy, the median proportion of CML cells among leukocytes is about 50%.

However, among the blood count values of CML patients under TKI therapy, the median white blood cell count (WBC), which is an indicator for CML, is 49.9 (normal range: 36.0-88.0 × 10^2/µL), and the median basophil percentage (BASO%) is 1.0 (normal range: 0-1.0%), both within the normal range.

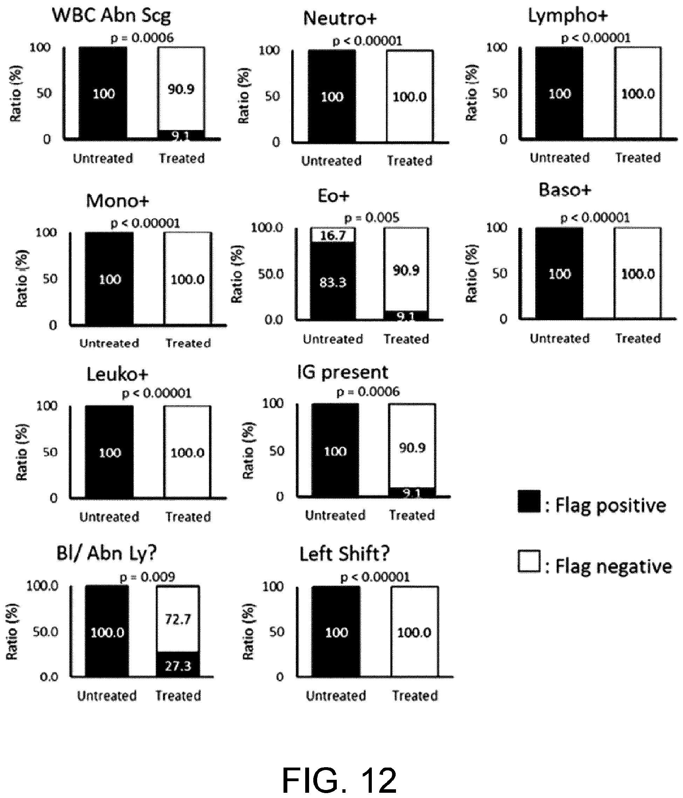

FIG. 12 is a graph showing whether an abnormal flag is raised (Flag positive) or not (Flag negative) for each sample when a blood count is performed by an automatic blood cell counter on the samples shown in FIG. 11.
The "Untreated" bar graph corresponds to the group of samples from CML patients before TKI therapy (n=6) in FIG. 11, and the "Treated" bar graph corresponds to the group of samples from CML patients under TKI therapy (n=11) in FIG. 11.
This graph shows that abnormal flags indicating characteristic findings suggestive of CML onset, such as increased WBC (leukocytes) or increased Baso (basophils), are rarely raised in CML patients under TKI therapy.

From the results of FIGS. 11 and 12, it is clear that even in samples where CML cells account for 50% or more of leukocytes in the blood, it is difficult to suspect CML by blood count using an automatic blood cell counter.
Therefore, in this verification experiment, CML patients under TKI therapy were assumed to be early-stage CML patients for whom screening is difficult by abnormal flags in blood count using an automatic blood cell counter, and it was verified whether discrimination by this method is possible.

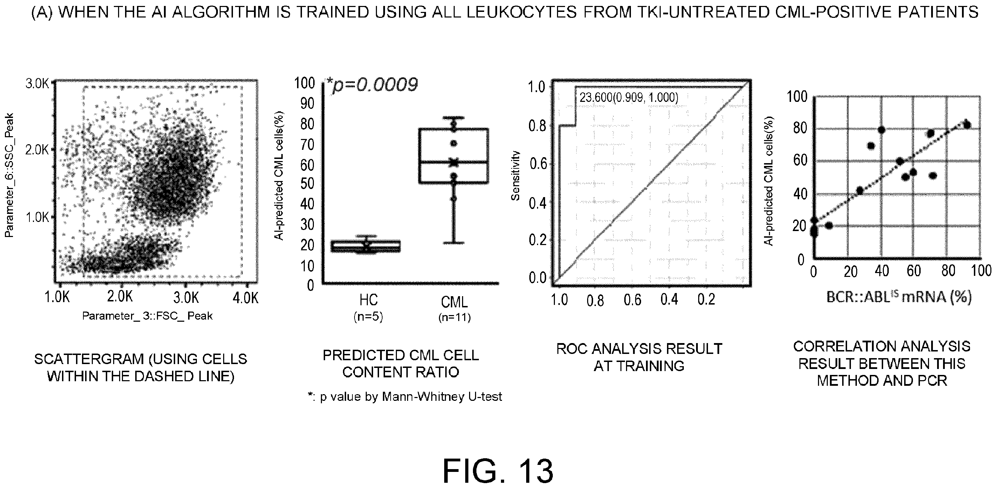

FIG. 13 is a verification example in which the AI algorithm 61 was trained (learned) using the waveform signals of all leukocytes from a CML-positive patient not treated with TKI as the "CML (+) waveform signal."

### <Learning Phase>

The left side of the figure shows a scattergram of a sample from a CML-positive patient not treated with TKI.
As described above, in this verification experiment, an optical system capable of acquiring flow cytometry signals in addition to the waveform signals of cells was used. In the scattergram, the horizontal axis represents the peak value of forward scattered light (peak intensity) obtained as a flow cytometry signal, and the vertical axis represents the peak value of side scattered light (peak intensity) obtained as a flow cytometry signal. In the scattergram, the cells within the dashed line were identified as leukocytes. The AI algorithm 61 was trained using the waveform signals of the identified leukocytes as the "CML (+) waveform signal." The AI algorithm 61 was also trained using the waveform signals of leukocytes contained in samples from healthy subjects as the "CML (-) waveform signal." In this way, the trained AI algorithm 62 was obtained.

### <Inference Phase>

For samples from healthy subjects (n=5) and samples assumed to be from early-stage CML patients, i.e., samples from CML patients under TKI therapy (n=11), waveform signals and flow cytometry signals of cells were obtained.
Leukocytes were identified based on the peak values of forward scattered light and side scattered light. The waveform signals of the identified leukocytes were input to the trained AI algorithm 62, and the content ratio of CML cells was calculated.

On the right side of the scattergram, the CML cell content ratio is shown for samples from healthy subjects (labeled "HC" in the figure) and for samples from the above hypothetical early-stage CML patients (labeled "CML" in the figure). This graph shows a significant difference in the CML cell content ratio among leukocytes between HC and CML, suggesting the possibility of early diagnosis of early-stage CML patients. To the right of that, the ROC curve at the time of training the AI algorithm 61 is shown. From this ROC curve, the AUC value is "0.98", indicating that the trained AI algorithm 62 is an effective model as a classification model.
On the far right of the figure, the results of a correlation analysis between the CML cell content ratio among leukocytes determined by this method and the Philadelphia chromosome BCR::ABL1 gene content ratio detected by PCR are shown. This figure shows a positive correlation between this method and the PCR results, demonstrating the effectiveness of this method.

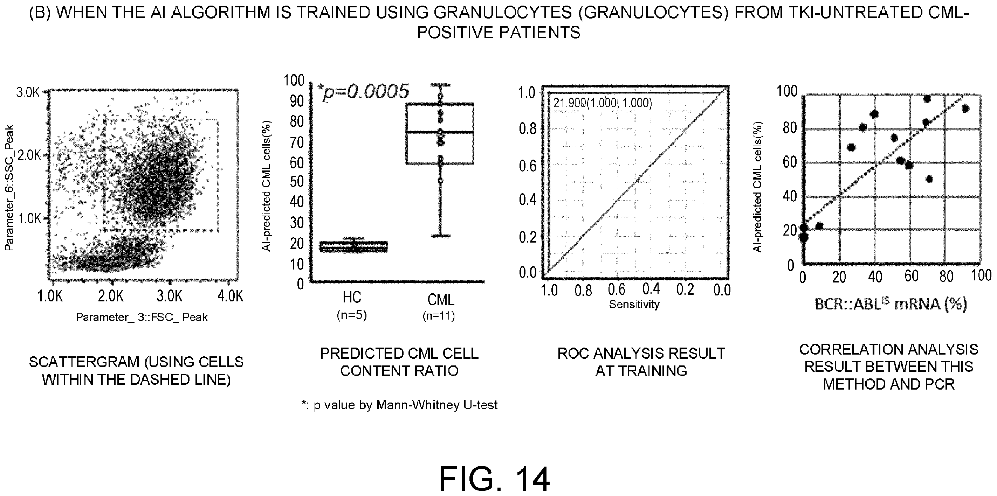

FIG. 14 is a verification example in which the AI algorithm 61 was trained (learned) using the waveform signals of granulocytes among the leukocytes of a CML-positive patient not treated with TKI as the "CML (+) waveform signal."
As in the verification example of FIG. 13, in order to match the population of cells to be analyzed in the learning and inference phases, the waveform signals of granulocytes were input to the AI algorithm 62 in the inference phase. Similarly, as the "CML (-) waveform signal", the waveform signals of granulocytes contained in samples from healthy subjects were used. The interpretation of each figure is the same as in FIG. 13.
These results show that when the AI algorithm 61 is trained using the waveform signals of granulocytes, there is no overlap in the CML cell content ratio among leukocytes between HC and CML, and a significant difference is observed. In addition, the AUC value is "1", indicating that the trained AI algorithm 62 is a very effective model as a classification model. In other words, training the AI algorithm 61 focusing on granulocytes strongly suggests the possibility of early diagnosis of early-stage CML patients.

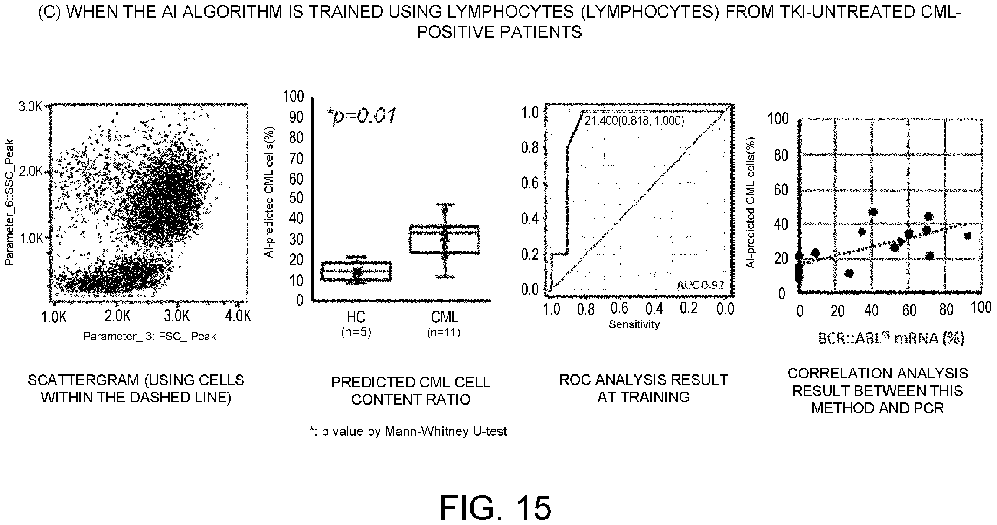

FIG. 15 is a verification example in which the AI algorithm 61 was trained (learned) using the waveform signals of lymphocytes among the leukocytes of a CML-positive patient not treated with TKI as the "CML (+) waveform signal."
As in the verification example of FIG. 13, in order to match the population of cells to be analyzed in the learning and inference phases, the waveform signals of lymphocytes were input to the AI algorithm 62 in the inference phase. Similarly, as the "CML (-) waveform signal", the waveform signals of lymphocytes contained in samples from healthy subjects were used. The interpretation of each figure is the same as in FIG. 13.
When the AI algorithm 61 is trained focusing on lymphocytes, the AUC value decreases to "0.92", but considering the property that lymphocytes can withstand long-term storage, the results suggest that even samples that have elapsed time since blood collection may help in the early diagnosis of CML.

From the results of the verification experiments shown in FIGS. 13 to 15, it was suggested that according to this method, CML cells in blood can be identified and early-stage CML patients, for whom screening is difficult by blood cell counting tests, can be accurately discriminated.
The reason why CML cells can be identified by GCM is considered to be that GCM can examine morphological features specific to CML cells in more detail than flow cytometry signals used in blood cell counting tests.
The present inventors have revealed that the BCR::ABL fusion gene characteristic of CML cells changes the morphology of mitochondria in the cells and causes excessive fragmentation of mitochondria.
According to GCM, it is possible to distinguish between normal cells and CML cells by comprehensively capturing subtle morphological changes caused by tumorigenesis, such as changes in mitochondrial morphology within cells, which are difficult to detect by conventional flow cytometry, and this is considered to have led to the results shown above.

In the above verification experiments, an optical system capable of acquiring flow cytometry signals in addition to GMI waveform signals was used to identify specific types of cells (e.g., leukocytes, granulocytes, lymphocytes), but a configuration for acquiring flow cytometry signals is not necessarily required, and this method can be implemented using only GMI waveform signals.
For example, in addition to the AI algorithm trained with CML waveform signals, an AI algorithm (type identification AI) that determines whether a cell is a specific type of cell (e.g., leukocyte) based on the waveform signal may be used.
In this case, for example, in the learning phase, the AI algorithm 61 can be trained by inputting the waveform signals of cells identified as leukocytes by the type identification AI among the cells of CML-positive patients not treated with TKI as CML (+) waveform signals.
In the inference phase, the discrimination result can be obtained by inputting the waveform signals of cells identified as leukocytes by the type identification AI among the cells of the patient sample to the trained AI algorithm 62.
Alternatively, learning and inference may be performed using the waveform signals of all particles in the sample from which waveform signals were obtained, without performing gating as shown in the scattergrams of FIGS. 13 to 15.
As can be seen from the scattergram in FIG. 13, since most of the particles in blood hemolyzed by a hemolytic agent are leukocytes, it is possible to analyze substantially only leukocytes even without gating.

### <Effects of the First Embodiment>

According to this embodiment, in the sample analyzer (for example, sample analyzer 1A), the measurement part (for example, GCM measurement unit 20) irradiates cells contained in the sample (for example, blood) with a plurality of diffracted lights generated by incident light on a diffractive optical element (for example, diffractive optical element 215) to acquire optical information (for example, GMI waveform signal (waveform information)).
Then, the analysis part (for example, control unit 30) analyzes the optical information obtained by the measurement part using an artificial intelligence algorithm (for example, the trained AI algorithm 62) to acquire information on leukemia cells contained in the sample (for example, whether it is a leukemia cell, the proportion of leukemia cells, the number of leukemia cells).
Thus, by analyzing the optical information obtained by the measurement part using an artificial intelligence algorithm, information on leukemia cells contained in the sample can be easily and appropriately obtained. That is, there is a possibility that CML can be detected with high accuracy by a simple blood test in the early stage of CML onset, leading to early diagnosis of CML.

Further, according to this embodiment, the analysis part acquires, as information on leukemia cells, the proportion of leukemia cells among leukocytes (for example, CML cell content ratio).
Thus, by analyzing the optical information obtained by the measurement part using an artificial intelligence algorithm, the proportion of leukemia cells among leukocytes contained in the sample can be easily and appropriately obtained.

Further, according to this embodiment, the analysis part acquires, as information on leukemia cells, the number of leukemia cells (for example, CML cell count).
Thus, by analyzing the optical information obtained by the measurement part using an artificial intelligence algorithm, the number of leukemia cells contained in the sample can be easily and appropriately obtained.

Further, according to this embodiment, the sample analyzer includes a sample preparator (for example, sample preparator 25) that prepares a measurement sample by mixing the sample and a reagent. The sample preparator prepares a measurement sample in which red blood cells contained in the sample are hemolyzed by using a hemolytic agent (for example, WDF hemolytic agent) as the reagent.
By using a hemolytic agent as the reagent, a measurement sample in which red blood cells contained in the sample are hemolyzed can be appropriately prepared.

In this case, the reagent may not contain a staining agent.
Thus, the measurement sample can be prepared by mixing the sample and a reagent not containing a staining agent. In addition, since the reagent does not contain a staining agent, information on leukemia cells contained in the sample can be obtained without labeling (no need for fluorescent labeling), and the optical system and light detector for collecting fluorescence can also be omitted.
Note that the reagent may contain a diluent instead of a staining agent.

Further, in this embodiment, the above artificial intelligence algorithm (for example, AI algorithm 62) is trained using, for example, optical information of leukocytes contained in a sample collected from a chronic myeloid leukemia patient (for example, waveform signals of all leukocytes from a CML-positive patient not treated with TKI) as training data.
By training the artificial intelligence algorithm with optical information of leukocytes contained in a sample collected from a chronic myeloid leukemia patient as training data, information on leukemia cells contained in the sample can be appropriately obtained.

Further, according to this embodiment, the above artificial intelligence algorithm (for example, AI algorithm 62) is trained using, for example, optical information of granulocytes contained in a sample collected from a chronic myeloid leukemia patient (for example, waveform signals of granulocytes from a CML-positive patient not treated with TKI) as training data.
By training the artificial intelligence algorithm with optical information of granulocytes contained in a sample collected from a chronic myeloid leukemia patient as training data, information on leukemia cells contained in the sample can be appropriately obtained.

### <Second Embodiment>

In the first embodiment, the sample was measured and CML cells were discriminated based on Ghost Cytometry by the GCM measurement unit 20.
The second embodiment relates to an embodiment in which the sample is preliminarily measured using flow cytometry, which has higher throughput than Ghost Cytometry, and Ghost Cytometry is applied to screened samples (samples suspected of CML) to discriminate CML cells.

The contents described in the second embodiment can be similarly applied to any of the other embodiments and modifications.

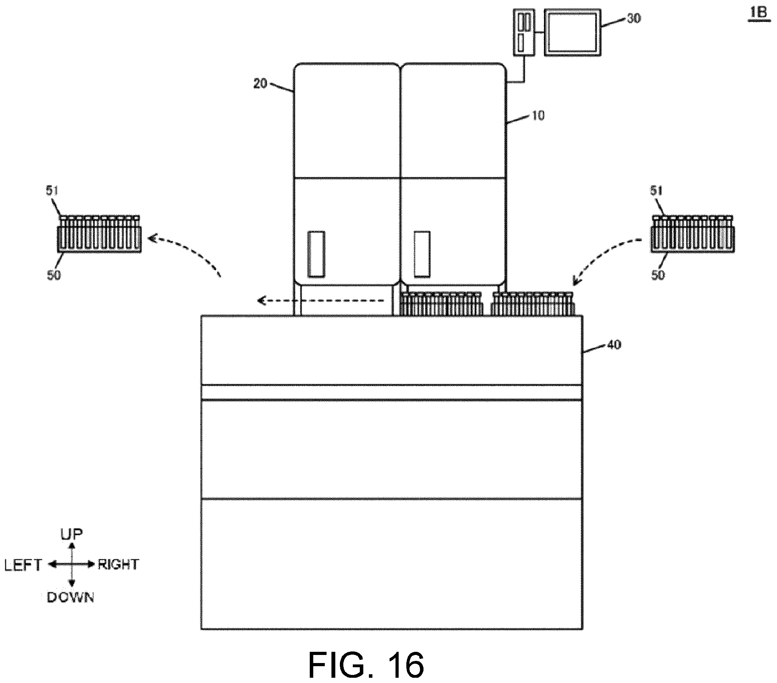

FIG. 16 is a front view schematically showing the configuration of a sample analyzer 1B as an example of the sample analyzer in the second embodiment.
The sample analyzer 1B includes, for example, a flow cytometry measurement unit (hereinafter referred to as "FCM measurement unit") 10, a GCM measurement unit 20, a control unit 30, and a conveyer 40.

The operator of the sample analyzer 1B, such as a laboratory technician, sets the sample container 51 containing the sample into the sample rack 50 and places the sample rack 50 on the right end region of the conveyer 40. The conveyer 40 transports the sample rack 50 and positions it as appropriate in front of the FCM measurement unit 10 and the GCM measurement unit 20.

The FCM measurement unit 10 takes out the sample container 51 from the sample rack 50, transfers it into the FCM measurement unit 10, and measures the sample in the sample container 51. When the measurement of the sample in the sample container 51 is completed, the FCM measurement unit 10 returns the sample container 51 to its original position in the sample rack 50.
Similarly, the GCM measurement unit 20 takes out the sample container 51 from the sample rack 50, transfers it into the GCM measurement unit 20, and measures the sample in the sample container 51. When the measurement of the sample in the sample container 51 is completed, the GCM measurement unit 20 returns the sample container 51 to its original position in the sample rack 50.
When all necessary measurements for all sample containers 51 on one sample rack 50 are completed, the conveyer 40 transports the sample rack 50 to the left end region of the conveyer 40. The laboratory technician removes the sample rack 50 transported to the left end region.

The FCM measurement unit 10 and the GCM measurement unit 20 are configured to be able to measure samples transported on the conveyer 40.
The conveyer 40 is configured to automatically supply the sample rack 50 containing the sample to the FCM measurement unit 10 and the GCM measurement unit 20.
Since the sample can be automatically supplied to the FCM measurement unit 10 and the GCM measurement unit 20 via the conveyer 40, the labor required by the laboratory technician to transfer the sample between the FCM measurement unit 10 and the GCM measurement unit 20 can be reduced.

The control unit 30 controls, for example, the FCM measurement unit 10, the GCM measurement unit 20, and the conveyer 40. The control unit 30 also analyzes, for example, the measurement information obtained by the FCM measurement unit 10 and the GCM measurement unit 20.

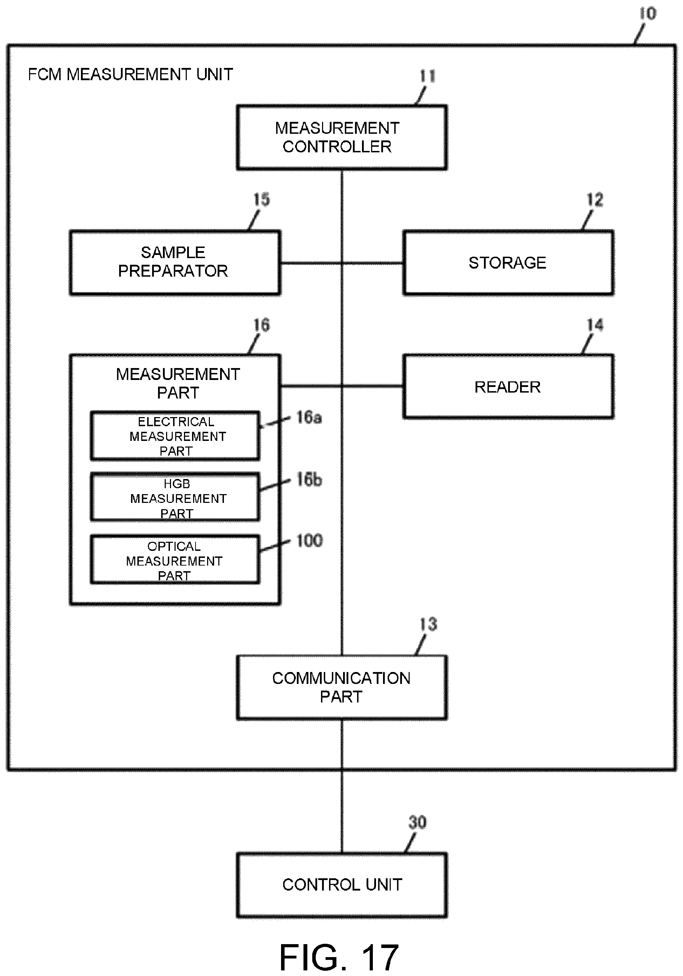

FIG. 17 is a block diagram showing an example of the functional configuration of the FCM measurement unit 10.

The FCM measurement unit 10 includes, for example, a measurement controller 11, storage 12, communication part 13, reader 14, sample preparator 15, and measurement part 16.

The measurement controller 11 includes, for example, an FPGA or CPU.
The storage 12 includes, for example, an HDD, SSD, RAM, or ROM.
The measurement controller 11 performs various processes based on a program stored in the storage 12 and controls each part of the FCM measurement unit 10.
The communication part 13 includes, for example, a connection terminal based on the USB standard, and communicates with the control unit 30.

The reader 14 includes, for example, a barcode reader. The reader 14 reads a barcode from a barcode label attached to the sample container 51 and acquires a sample ID.
The sample preparator 15 aspirates a sample from the sample container 51 and mixes a reagent with the aspirated sample to prepare a measurement sample.

The measurement part 16 includes, for example, an electrical measurement part 16a, an HGB measurement part 16b (HGB: hemoglobin), and an optical measurement part 100.
The electrical measurement part 16a measures cells (blood cells) in the sample by, for example, the sheath flow DC detection method.
The HGB measurement part 16b measures hemoglobin in cells (blood cells) in the sample by, for example, the SLS-hemoglobin method.
The optical measurement part 100 measures cells (blood cells) in the sample by, for example, the flow cytometry method.

The electrical measurement part 16a and the HGB measurement part 16b include, for example, an amplifier and an A/D converter, perform signal processing on the detection signal obtained by measurement, and output the processed measurement information to the measurement controller 11.

The optical measurement part 100 includes, for example, an amplifier and an A/D converter, performs signal processing on the detection signal obtained by measurement, and outputs the processed measurement data to the measurement controller 11.

The measurement controller 11 stores the measurement data output from the measurement part 16 in the storage 12. When the measurement of one sample is completed, the measurement controller 11 transmits the measurement data stored in the storage 12 to the control unit 30 in association with the sample ID read by the reader 14.

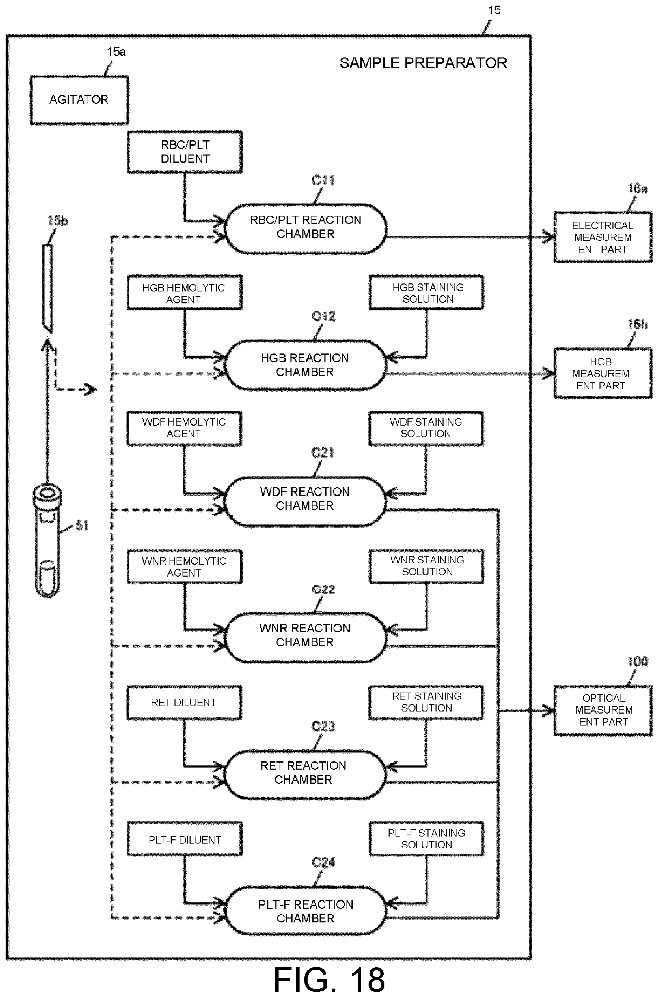

FIG. 18 is a block diagram showing an example of the functional configuration of the sample preparator 15 configured to prepare a measurement sample.

The sample preparator 15 includes, for example, an agitator 15a, an aspiration pipette 15b, and reaction chambers C11, C12, and C21-C24.

The agitator 15a is configured to grip the sample container 51 and to agitate the sample in the gripped sample container 51 by oscillating it.
The aspiration pipette 15b is, for example, a nozzle with a pointed lower end, and is configured to penetrate the lid of the sample container 51 made of an elastic material. The aspiration pipette 15b aspirates the sample from the sample container 51 after agitation and dispenses the aspirated sample as appropriate into the reaction chambers C11, C12, and C21-C24.

In the reaction chamber C11, the sample and RBC/PLT diluent are mixed to prepare an RBC/PLT measurement sample. The RBC/PLT diluent is, for example, CELLPACK^{®} DCL. The RBC/PLT measurement sample prepared in the reaction chamber C11 is measured by the electrical measurement part 16a.
The electrical measurement part 16a acquires a detection signal corresponding to blood cells in the RBC/PLT measurement sample, performs signal processing on the acquired detection signal, and obtains measurement information. The controller 31 (see FIG. 7) of the control unit 30 analyzes the measurement information obtained by measuring the RBC/PLT measurement sample and obtains the red blood cell count, platelet count, etc.

In the reaction chamber C12, the sample, HGB hemolytic agent, and HGB diluent are mixed to prepare an HGB measurement sample. The HGB hemolytic agent is, for example, SULFOLYSER^{®}, and the HGB diluent is, for example, CELLPACK^{®} DCL. The HGB measurement sample prepared in the reaction chamber C12 is measured by the HGB measurement part 16b.
The HGB measurement part 16b acquires a detection signal corresponding to the hemoglobin concentration, performs signal processing on the acquired detection signal, and obtains measurement information. The controller 31 of the control unit 30 analyzes the measurement information obtained by measuring the HGB measurement sample and obtains the hemoglobin concentration, etc.

In the reaction chamber C21, the sample, WDF hemolytic agent, and WDF staining solution are mixed to prepare a WDF measurement sample. The WDF hemolytic agent is, for example, LYSERCELL^{®} WDFII, and the WDF staining solution is, for example, FLUOROCELL^{®} WDF. The WDF measurement sample prepared in the reaction chamber C21 is measured by the optical measurement part 100.
The optical measurement part 100 acquires a detection signal corresponding to blood cells in the WDF measurement sample, performs signal processing on the acquired detection signal, and obtains measurement data. The controller 31 of the control unit 30 analyzes the measurement data obtained by measuring the WDF measurement sample and the measurement data obtained by measuring the WNR measurement sample described later, classifies neutrophils, normal lymphocytes, monocytes, eosinophils, basophils, blasts, abnormal lymphocytes, atypical lymphocytes, immature granulocytes, nucleated red blood cells, etc., and obtains the number of each blood cell.

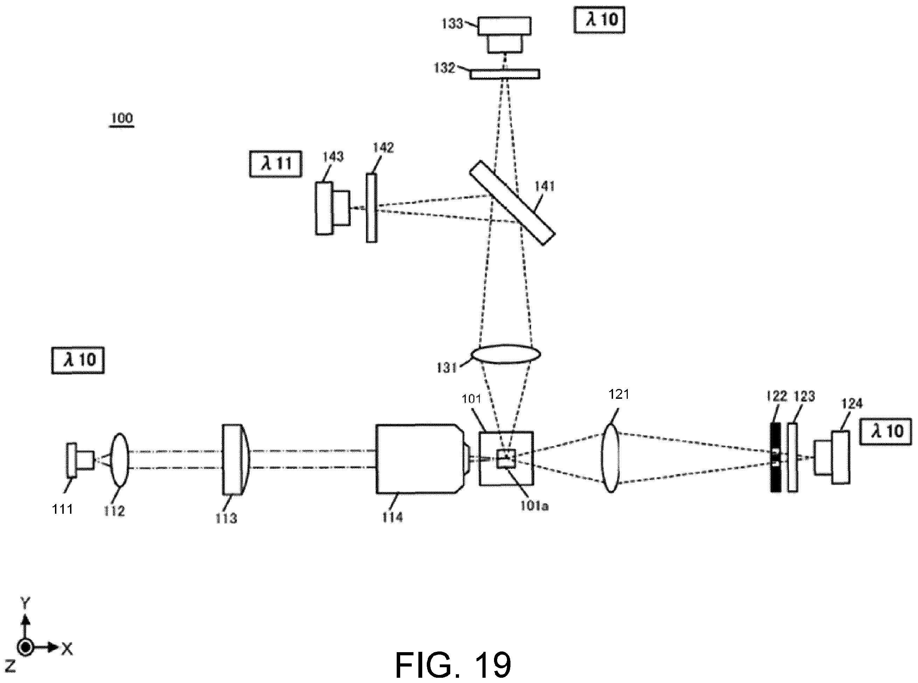

In this case, the measurement data includes time-series data of side scattered light corresponding to each cell, indicating the intensity change of side scattered light received by the light detector 133 while each cell in the WDF measurement sample flowing through the flow cell 101 (see FIG. 19) passes through the beam spot BS, and time-series data of fluorescence corresponding to each cell, indicating the intensity change of fluorescence received by the light detector 143 while each cell in the WDF measurement sample flowing through the flow cell 101 passes through the beam spot BS. The controller 31 of the control unit 30 obtains the peak values of side scattered light and fluorescence corresponding to each cell from the time-series data of side scattered light and fluorescence, and generates a scattergram.

In the reaction chamber C22, the sample, WNR hemolytic agent, and WNR staining solution are mixed to prepare a WNR measurement sample. The WNR hemolytic agent is, for example, LYSERCELL^{®} WNR, and the WNR staining solution is, for example, FLUOROCELL^{®} WNR. The WNR measurement sample prepared in the reaction chamber C22 is measured by the optical measurement part 100.
The optical measurement part 100 acquires a detection signal corresponding to blood cells in the WNR measurement sample, performs signal processing on the acquired detection signal, and obtains measurement data. The controller 31 of the control unit 30 analyzes the measurement data obtained by measuring the WNR measurement sample, classifies leukocytes and nucleated red blood cells, etc., and obtains the number of each blood cell.

In this case, the measurement data includes time-series data of forward scattered light corresponding to each cell, indicating the intensity change of forward scattered light received by the light detector 124 while each cell in the WNR measurement sample flowing through the flow cell 101 (see FIG. 19) passes through the beam spot BS, and time-series data of fluorescence corresponding to each cell, indicating the intensity change of fluorescence received by the light detector 143 while each cell in the WNR measurement sample flowing through the flow cell 101 passes through the beam spot BS. The controller 31 of the control unit 30 obtains the peak values of forward scattered light and fluorescence corresponding to each cell from the time-series data of forward scattered light and fluorescence, and generates a scattergram.

In the reaction chamber C23, the sample, RET diluent, and RET staining solution are mixed to prepare a RET measurement sample. The RET diluent is, for example, CELLPACK^{®} DFL, and the RET staining solution is, for example, FLUOROCELL^{®} RET. The RET measurement sample prepared in the reaction chamber C23 is measured by the optical measurement part 100.
The optical measurement part 100 acquires a detection signal corresponding to blood cells in the RET measurement sample, performs signal processing on the acquired detection signal, and obtains measurement data. The controller 31 of the control unit 30 analyzes the measurement data obtained by measuring the RET measurement sample, classifies reticulocytes, etc., and obtains the number of each blood cell.

In this case, the measurement data includes time-series data of forward scattered light corresponding to each cell, indicating the intensity change of forward scattered light received by the light detector 124 while each cell in the RET measurement sample flowing through the flow cell 101 (see FIG. 19) passes through the beam spot BS, and time-series data of fluorescence corresponding to each cell, indicating the intensity change of fluorescence received by the light detector 143 while each cell in the RET measurement sample flowing through the flow cell 101 passes through the beam spot BS. The controller 31 of the control unit 30 obtains the peak values of forward scattered light and fluorescence corresponding to each cell from the time-series data of forward scattered light and fluorescence, and generates a scattergram.

In the reaction chamber C24, the sample, PLT-F diluent, and PLT-F staining solution are mixed to prepare a PLT-F measurement sample. The PLT-F diluent is, for example, CELLPACK^{®} DFL, and the PLT-F staining solution is, for example, FLUOROCELL^{®} PLT. The PLT-F measurement sample prepared in the reaction chamber C24 is measured by the optical measurement part 100.
The optical measurement part 100 acquires a detection signal corresponding to blood cells in the PLT-F measurement sample, performs signal processing on the acquired detection signal, and obtains measurement data. The controller 31 of the control unit 30 analyzes the measurement data obtained by measuring the PLT-F measurement sample, classifies platelets, etc., and obtains the number of each blood cell.

In this case, the measurement data includes time-series data of forward scattered light corresponding to each cell, indicating the intensity change of forward scattered light received by the light detector 124 while each cell in the PLT-F measurement sample flowing through the flow cell 101 (see FIG. 19) passes through the beam spot BS, and time-series data of fluorescence corresponding to each cell, indicating the intensity change of fluorescence received by the light detector 143 while each cell in the PLT-F measurement sample flowing through the flow cell 101 passes through the beam spot BS. The controller 31 of the control unit 30 obtains the peak values of forward scattered light and fluorescence corresponding to each cell from the time-series data of forward scattered light and fluorescence, and generates a scattergram.

Note that the measurement data may be any information reflecting the size, shape, internal structure, or nucleic acid content of each cell, obtained by irradiating each cell in the measurement sample with at least one light having a single beam spot, and is not limited to the above peak values.
In addition, the diluent, hemolytic agent, and staining solution mixed in the reaction chambers C11, C12, and C21-C24 are not limited to the above reagents.

FIG. 19 is a diagram schematically showing the configuration of the optical measurement part 100. For convenience, mutually orthogonal X, Y, and Z axes are shown in FIG. 19. The Z-axis direction is the flow direction of the measurement sample in the flow cell 101.

The optical measurement part 100 includes a flow cell 101, a light source 111, a collimator lens 112, a cylindrical lens 113, a condenser lens 114, condenser lenses 121 and 131, a beam stopper 122, optical filters 123, 132, and 142, light detectors 124, 133, and 143, and a dichroic mirror 141.

The light source 111 is, for example, a semiconductor laser light source. The light source 111 emits light of a predetermined wavelength λ10 in the X-axis direction. The wavelength λ10 is, for example, 488 nm or 642 nm.
The collimator lens 112 converts the light emitted from the light source 111 into parallel light.
The cylindrical lens 113 converges the light from the light source 111 in the Y-axis direction.
The condenser lens 114 converges the light from the light source 111 in the Y-axis and Z-axis directions, forms it into a flat shape at the position of the flow cell 101, and condenses it onto the flow path 101a of the flow cell 101.

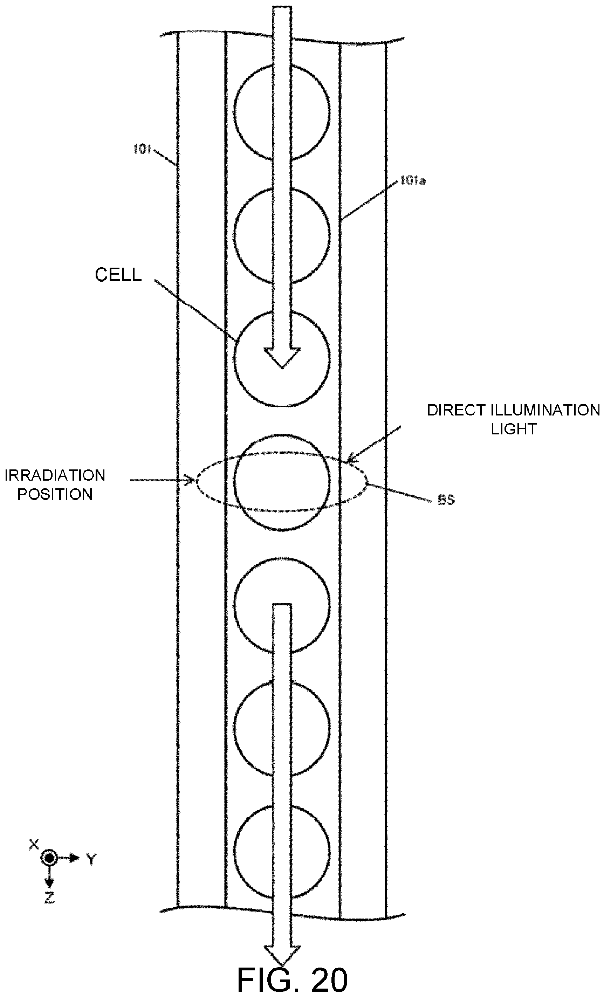

FIG. 20 is a side view schematically showing the configuration of the flow cell 101.

The light from the light source 111 is irradiated onto the irradiation position of the flow path 101a of the flow cell 101 as a single beam spot BS having a flat shape with a small width in the Z-axis direction, by the function of the cylindrical lens 113 and the condenser lens 114.
Hereinafter, the light emitted from the light source 111 and irradiated onto the irradiation position of the flow path 101a is referred to as "direct illumination light."
When the direct illumination light is irradiated onto the cells flowing through the flow path 101a, forward scattered light, side scattered light, and fluorescence are generated from the portions of the cells irradiated with the light. Here, it is assumed that when the direct illumination light of wavelength λ10 is irradiated onto the fluorescent dye staining the cells, light of wavelength λ11 is generated from the fluorescent dye.

As described above, since the flow rate per unit time is adjusted to be substantially constant by the fluid adjustment part 200a of the flow cell 201, the throughput of measurement by the GCM measurement unit 20 may be lower than the throughput of measurement by the FCM measurement unit 10. In addition, the controller 31 can control the operation of the GCM measurement unit 20 so that the number of cells measured by the GCM measurement unit 20 is less than the number of cells measured by the FCM measurement unit 10, according to the flow rate adjustment.

Returning to FIG. 19, the condenser lens 121 condenses the forward scattered light of wavelength λ10 generated from the cells onto the light detector 124. The beam stopper 122 blocks the light of wavelength λ10 that has passed through the flow cell 101 without being irradiated onto the cells, and allows the forward scattered light of wavelength λ10 generated from the cells to pass through. The optical filter 123 is configured to transmit only light of wavelength λ10. The light detector 124 receives the forward scattered light of wavelength λ10 transmitted through the optical filter 123 and outputs a detection signal according to the received intensity. The light detector 124 is, for example, a photodiode (PD).

The condenser lens 131 condenses the side scattered light of wavelength λ10 generated from the cells onto the light detector 133, and condenses the fluorescence of wavelength λ11 generated from the cells onto the light detector 143. The dichroic mirror 141 transmits light of wavelength λ10 and reflects light of wavelength λ11. The optical filter 132 is configured to transmit only light of wavelength λ10 from the dichroic mirror 141. The light detector 133 receives the side scattered light of wavelength λ10 transmitted through the optical filter 132 and outputs a detection signal according to the received intensity. The light detector 133 is, for example, a photodiode (PD).

The optical filter 142 is configured to transmit only light of wavelength λ11 from the dichroic mirror 141. The light detector 143 receives the fluorescence of wavelength λ11 transmitted through the optical filter 142 and outputs a detection signal according to the received intensity. The light detector 143 is, for example, a photomultiplier tube (PMT), avalanche photodiode (APD), or photodiode (PD).

The controller 31 analyzes cells based on the measurement information obtained by the FCM measurement unit 10 and the GCM measurement unit 20, for example.

The controller 31 analyzes the measurement information obtained by the electrical measurement part 16a and the HGB measurement part 16b of the FCM measurement unit 10 and the measurement data obtained by the optical measurement part 100 of the FCM measurement unit 10 to obtain FCM analysis results. For example, the controller 31 generates a scattergram for each sample based on the measurement data and classifies cells based on the generated scattergram.

For example, the controller 31 generates a scattergram based on the measurement data obtained by measuring the WDF measurement sample, and groups a plurality of cell populations corresponding to the plots on the generated scattergram. In grouping the cell populations, for example, the controller 31 calculates the centroid of the plots in a predetermined region on the scattergram, performs cluster analysis on the cell populations based on the distance from each plot to the centroid, and classifies each cell. Examples of scattergrams will be described later.

By grouping the cell populations, for example, regions corresponding to normal lymphocytes, monocytes, neutrophils and basophils, and eosinophils are set. In addition, if the measurement sample contains blasts, abnormal lymphocytes, atypical lymphocytes, immature granulocytes, or nucleated red blood cells, regions corresponding to these blood cells are also set. The controller 31 counts the plots in the regions set in the scattergram to obtain the number of blood cells in each classification.

Note that the controller 31 does not necessarily have to actually generate a scattergram and group the cell populations, and may perform grouping based on the cell populations by processing data corresponding to the scattergram, for example.

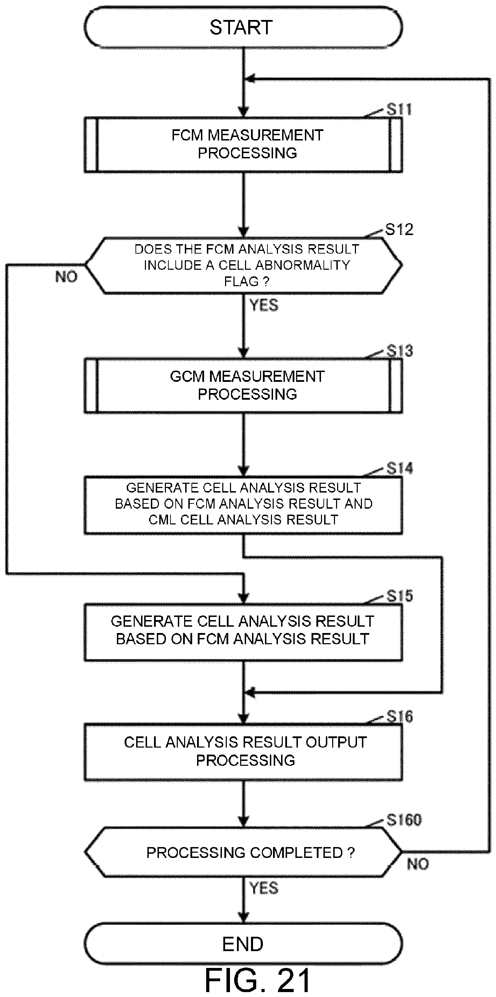

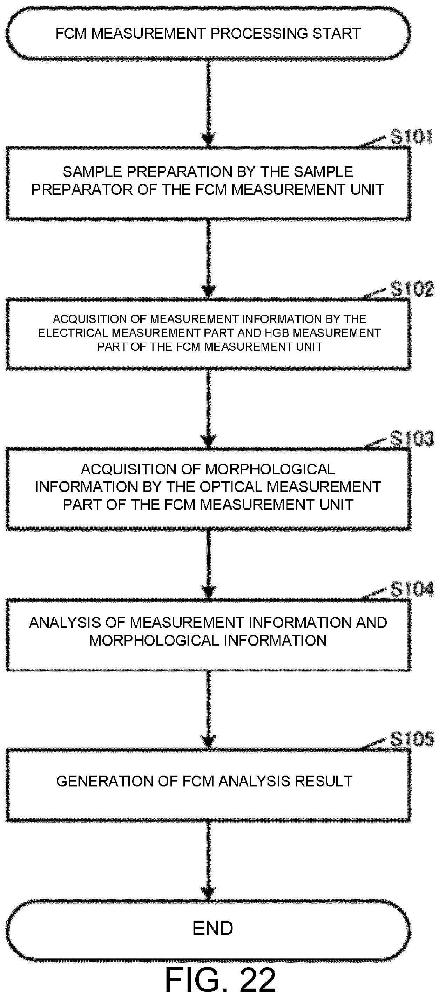

Next, with reference to FIGS. 21 and 22, the measurement processing of the sample analyzer 1B will be described.

FIG. 21 is a flowchart showing an example of the flow of control processing related to measurement by the control unit 30.

In step S11, the controller 31 of the control unit 30 controls the FCM measurement unit 10 so that FCM measurement processing is performed. As a result, the controller 31 analyzes the measurement information and measurement data obtained by the FCM measurement unit 10 and obtains FCM analysis results.

The FCM analysis results may include, for example, the count value of cells in the sample (number per unit volume, etc.), an abnormal cell flag indicating the presence of abnormal cells, and a scattergram or histogram.

The count values in the FCM analysis results may be, for example, the count values of red blood cells, white blood cells, neutrophils, normal lymphocytes, monocytes, eosinophils, basophils, platelets, abnormal cells, etc.
The abnormal cell flag in the FCM analysis results may include, for example, a flag indicating that the number per unit volume of abnormal cells such as blasts, abnormal lymphocytes, atypical lymphocytes, immature granulocytes, nucleated red blood cells, etc., in the sample is equal to or greater than a predetermined threshold, and a flag indicating that the classification state of white blood cells is abnormal. Abnormal cells may be defined as cells that are not present or are present in only small numbers in the peripheral blood of healthy individuals when the sample is blood. The appearance of abnormal cells may be considered when the number per unit volume of abnormal cells is equal to or greater than a predetermined threshold.

If the number per unit volume of abnormal cells such as blasts, abnormal lymphocytes, atypical lymphocytes, immature granulocytes, nucleated red blood cells, etc., in the sample does not meet the condition of being equal to or greater than the predetermined threshold, the FCM analysis results do not include an abnormal cell flag.

The FCM measurement processing will be described later with reference to FIG. 22.

In step S12, the controller 31 determines whether the FCM analysis results obtained in step S11 include an abnormal cell flag.
If it is determined that the FCM analysis results include an abnormal cell flag (step S12: YES), in step S13, the controller 31 controls the GCM measurement unit 20 so that GCM measurement processing is performed. As a result, the controller 31 analyzes the waveform signals obtained by the GCM measurement unit 20 and obtains GCM analysis results.

The GCM measurement processing can be performed, for example, according to steps S110 to S140 in FIG. 10.

Subsequently, in step S14, the controller 31 generates analysis results (cell analysis results) of the cells in the sample based on the FCM analysis results obtained in step S11 and the GCM analysis results obtained in step S13.

On the other hand, if it is determined that the FCM analysis results do not include an abnormal cell flag (step S12: NO), in step S15, the controller 31 generates analysis results (cell analysis results) of the cells in the sample based on the FCM analysis results obtained in step S11.

That is, in step S12, the controller 31 selectively determines whether to generate cell analysis results based on the FCM analysis results or to generate cell analysis results based on both the FCM analysis results and the GCM analysis results.

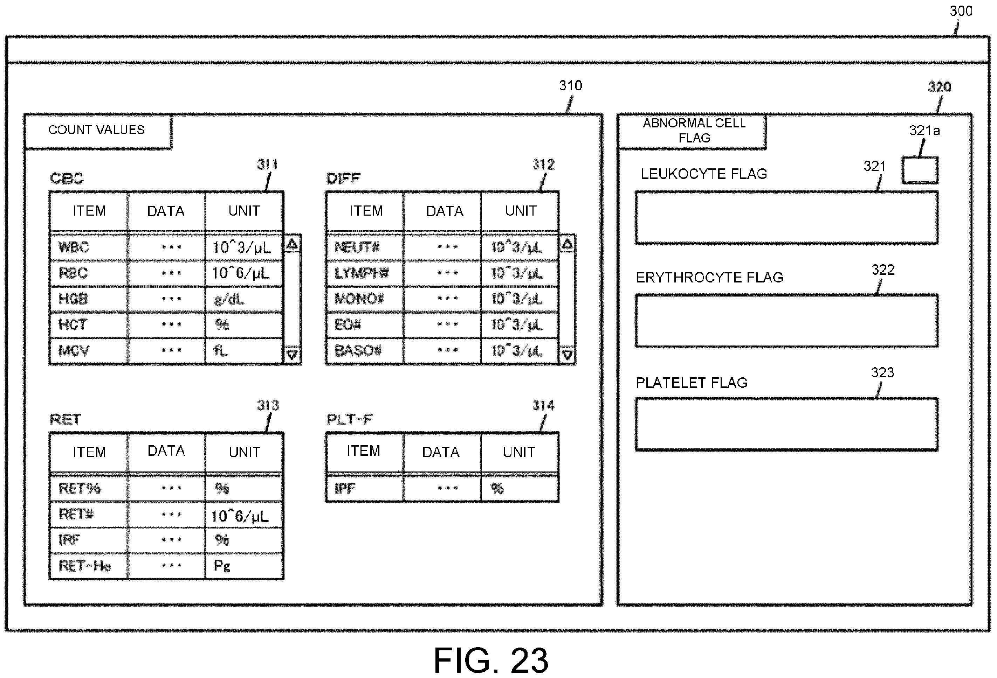

For example, when a laboratory technician inputs a display instruction via the input part 35, in step S16, the controller 31 displays the cell analysis result screen 300 including the cell analysis results generated in step S14 or step S15 on the display 34. The cell analysis result screen 300 will be described later with reference to FIGS. 23 to 30.

As described above, in the example of FIG. 21, since the GCM measurement processing is executed when the FCM analysis results include an abnormal cell flag, the measurement frequency by the GCM measurement unit 20 is lower than the measurement frequency by the FCM measurement unit 10.
That is, the controller 31 can control each measurement unit so that the measurement frequency by the GCM measurement unit 20 is lower than the measurement frequency by the FCM measurement unit 10.
As described with reference to FIG. 5, in the GCM measurement unit 20, the flow rate of the measurement sample is adjusted so that only one cell is positioned in the irradiation region R of the diffracted illumination light at a time. Therefore, the throughput of measurement by the GCM measurement unit 20 may be lower than the throughput of measurement by the FCM measurement unit 10.
Therefore, if the measurement frequency by the GCM measurement unit 20 becomes comparable to that by the FCM measurement unit 10, the overall throughput of the laboratory may decrease. By making the measurement frequency by the GCM measurement unit 20 lower than that by the FCM measurement unit 10, it is possible to take advantage of the GCM measurement unit 20 while suppressing a decrease in the overall throughput of the laboratory.

FIG. 22 is a flowchart showing an example of the flow of FCM measurement processing.

In step S101, the controller 31 of the control unit 30 controls the FCM measurement unit 10 so that the sample preparator 15 (see FIGS. 17 and 18) of the FCM measurement unit 10 prepares the measurement sample. As a result, measurement samples are prepared in the reaction chambers C11, C12, and C21-C24.

Here, for convenience, it is described as if measurement samples are prepared in all the reaction chambers C11, C12, and C21-C24, but in practice, the necessary measurement samples are prepared according to the measurement items specified for the target sample, and in subsequent steps S102 and S103, the measurement samples are measured by the electrical measurement part 16a, HGB measurement part 16b, and optical measurement part 100 according to the prepared measurement samples.

In step S102, the controller 31 controls the FCM measurement unit 10 so that measurement is performed by the electrical measurement part 16a and the HGB measurement part 16b, and acquires measurement information based on this measurement from the FCM measurement unit 10.

In step S103, the controller 31 controls the FCM measurement unit 10 so that measurement is performed by the optical measurement part 100. As a result, as shown in FIG. 20, the optical measurement part 100 irradiates the cells in the sample contained in the measurement sample flowing through the flow cell 101 with direct illumination light having a single beam spot BS and acquires measurement data. The controller 31 then acquires the measurement data obtained by the measurement part 16 from the FCM measurement unit 10.

In step S104, the controller 31 analyzes the measurement information acquired in step S102 and the measurement data acquired in step S103.

In step S105, the controller 31 generates FCM analysis information based on the analysis in step S104. The FCM analysis information includes the count values of blood cells obtained by analyzing the measurement information based on the measurements by the electrical measurement part 16a and the HGB measurement part 16b, and the count values of blood cells and abnormal cell flags obtained by analyzing the measurement data based on the measurement by the optical measurement part 100.

Next, with reference to FIGS. 23 to 25, an example of the cell analysis result screen 300 displayed in step S16 of FIG. 21 will be described.

FIG. 23 is a diagram schematically showing the configuration of the cell analysis result screen 300.

The cell analysis result screen 300 includes, for example, a count value display region 310 and an abnormal cell flag display region 320.

The count value display region 310 includes display regions 311 to 314 respectively corresponding to the analysis modes of CBC, DIFF, RET, and PLT-F, for example.

In the display region 311, count values corresponding to the CBC mode, such as white blood cell count, red blood cell count, hemoglobin amount, hematocrit value, mean corpuscular volume, etc., are displayed.

In the display region 312, count values corresponding to the DIFF mode, such as neutrophil count, lymphocyte count, monocyte count, eosinophil count, basophil count, etc., are displayed.

In the display region 313, count values corresponding to the RET mode, such as reticulocyte ratio, reticulocyte count, immature reticulocyte fraction, reticulocyte hemoglobin equivalent, etc., are displayed.

In the display region 314, count values corresponding to the PLT-F mode, such as immature platelet ratio, are displayed.

Note that the scattergram or histogram included in the FCM analysis results may be displayed on the cell analysis result screen 300.

The abnormal cell flag display region 320 includes display regions 321 to 323 to display abnormal cell flags for leukocytes, erythrocytes, and platelets, respectively.

In the display region 321, a label 321a is attached, and the label 321a displays the number 1 or 2. A label 321a displaying "1" indicates that the abnormal cell flag for leukocytes displayed in the display region 321 is based on the FCM analysis results, and a label 321a displaying "2" indicates that the abnormal cell flag for leukocytes displayed in the display region 321 is based on the GCM analysis results.

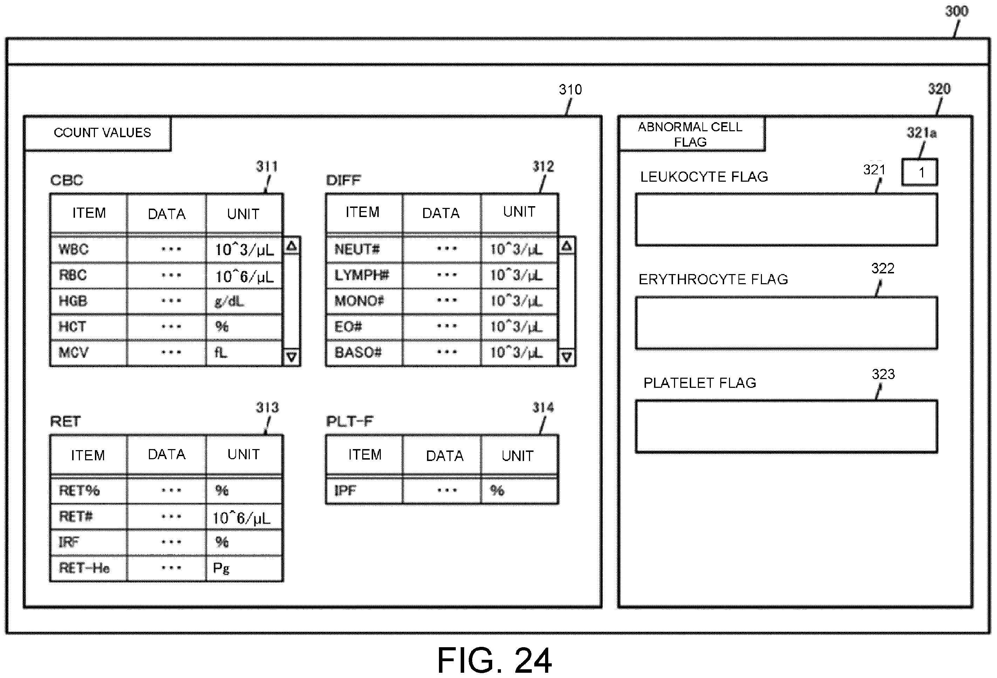

When step S15 in FIG. 21 is executed, since the GCM measurement processing is not performed, the GCM analysis result is not obtained, and the cell analysis result is generated based on the FCM analysis result from the FCM measurement processing. Therefore, in this case, as shown in FIG. 24, the cell analysis result screen 300 displays the cell analysis result based only on the FCM analysis result, and the label 321a displays "1" in the display region 321 for abnormal cell flags for leukocytes to indicate that the abnormal cell flag is based on the FCM analysis result. In the example shown in FIG. 23, since there is no abnormal cell flag based on the FCM analysis result, no abnormal cell flag is displayed in the display regions 321 to 323.

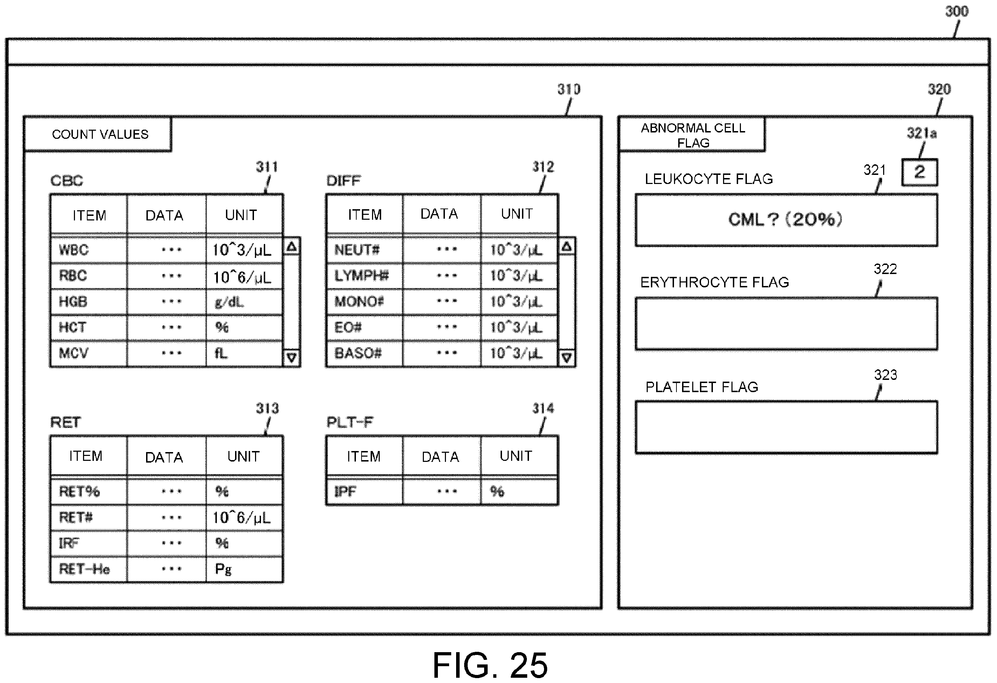

When step S14 in FIG. 21 is executed, the GCM measurement processing is performed, and the cell analysis result is generated based on both the FCM analysis result from the FCM measurement processing and the GCM analysis result. In this case, as shown in FIG. 25, in the cell analysis result screen 300, the count value display region 310 and the display regions 322 and 323 display count values and abnormal cell flags based on the FCM analysis result, and the display region 321 for abnormal cell flags for leukocytes displays the result based on the GCM analysis result instead of the abnormal cell flag based on the FCM analysis result. The label 321a displays "2" to indicate that the GCM analysis result is displayed in the display region 321. In the example shown in FIG. 25, the display region 321 displays "CML?" indicating suspicion of CML and "CML cell content ratio (20%)" based on the GCM analysis result. That is, in this example, at least part of the result based on the FCM analysis result is supplemented by the GCM analysis result. For example, the display of "CML?" may be shown when the CML cell content ratio exceeds a predetermined ratio (for example, "20%") or is equal to or greater than the predetermined ratio. The CML cell content ratio may not be displayed, or only the CML cell content ratio may be displayed.

Note that when the GCM measurement processing is performed and the GCM analysis result indicates a low suspicion of CML (for example, the CML cell content ratio is less than or equal to a predetermined ratio), "CML?" as a comparative example is not displayed in the display region 321, and "2" may be displayed in the label 321a.

In this way, regardless of the presence or type of abnormal flag in the FCM analysis result, when the GCM measurement processing is performed, the display is based on the GCM analysis result.
As a result, the laboratory technician can accurately grasp the presence of abnormal cells in the target sample, so the necessity of preparing a smear specimen can be accurately determined in the subsequent stage of the sample analyzer 1B, and even when preparing a smear specimen, the confirmation of the smear specimen can be smoothly performed based on the accurate abnormal cell flag based on the GCM analysis result.

### <Effects of the Second Embodiment>

In this embodiment, the sample analyzer (for example, sample analyzer 1B) further includes a first measurement part (for example, FCM measurement unit 10) that acquires measurement results regarding information on blood cells contained in the sample. The above-mentioned measurement part is a second measurement part (for example, GCM measurement unit 20) different from the first measurement part, and the second measurement part performs measurement of the sample and acquires optical information of cells contained in the sample when the measurement result by the first measurement part for the sample satisfies a predetermined condition (for example, the number per unit volume of abnormal cells such as blasts, abnormal lymphocytes, atypical lymphocytes, immature granulocytes, nucleated red blood cells, etc., in the sample is equal to or greater than a predetermined threshold).
According to this, when the measurement result by the first measurement part for the sample satisfies the predetermined condition, the second measurement part different from the first measurement part performs measurement of the sample and acquires optical information of cells contained in the sample. As a result, the measurement frequency of the second measurement part becomes lower than that of the first measurement part, so that, for example, as described above, a decrease in throughput can be suppressed.

In this case, the predetermined condition may include an increase in a specific type of leukocyte or the appearance of blasts.
According to this, when the measurement result by the first measurement part for the sample satisfies the condition of an increase in a specific type of leukocyte or the appearance of blasts, the second measurement part performs measurement of the sample and acquires optical information of cells contained in the sample.
When an increase in a specific type of leukocyte or the appearance of blasts is observed, CML may be suspected. Therefore, the second measurement part can perform measurement of the sample and acquire optical information of cells contained in the sample with setting an increase in basophils or the appearance of blasts as the predetermined condition.

In this case, the specific type of leukocyte may be basophils.
According to this, when the measurement result by the first measurement part for the sample satisfies the condition of an increase in basophils or the appearance of blasts, the second measurement part performs measurement of the sample and acquires optical information of cells contained in the sample.
When an increase in basophils is observed, CML may be suspected. Therefore, the second measurement part can perform measurement of the sample and acquire optical information of cells contained in the sample with setting an increase in basophils as the predetermined condition.

Note that, instead of blasts, the predetermined condition may include the appearance of abnormal cells such as abnormal lymphocytes, atypical lymphocytes, immature granulocytes, or nucleated red blood cells.

### <Modification of the Second Embodiment>

In the above embodiment, when the GCM measurement processing is performed, not only the GCM analysis result but also the FCM analysis result is displayed as the cell analysis result. However, the FCM analysis result does not necessarily have to be displayed.

For example, in step S14 of FIG. 21, the controller 31 of the control unit 30 may generate the cell analysis result based only on the GCM analysis result. The configuration of the cell analysis result screen 300 when the GCM measurement processing is performed may be the same as the cell analysis result screen 600 in FIG. 9, for example.

In this modification, according to the GCM analysis result, the number of samples resulting in false positives can be reduced, so the number of times for preparing and checking smear specimens and the like can be reduced, and more accurate cell analysis results can be used as a reference for checking smear specimens and the like, and the preparation and checking of smear specimens can be omitted. Therefore, the burden on the laboratory technician can be reduced.

### <Third Embodiment>

In the second embodiment, the FCM measurement processing and GCM measurement processing were performed by the FCM measurement unit 10 and the GCM measurement unit 20, respectively.
In contrast, the third embodiment relates to an embodiment in which both the FCM measurement processing and GCM measurement processing are performed by a flow cytometry/ghost cytometry integrated measurement unit (hereinafter referred to as "integrated measurement unit").

The contents described in the third embodiment can be similarly applied to any of the other embodiments and modifications.

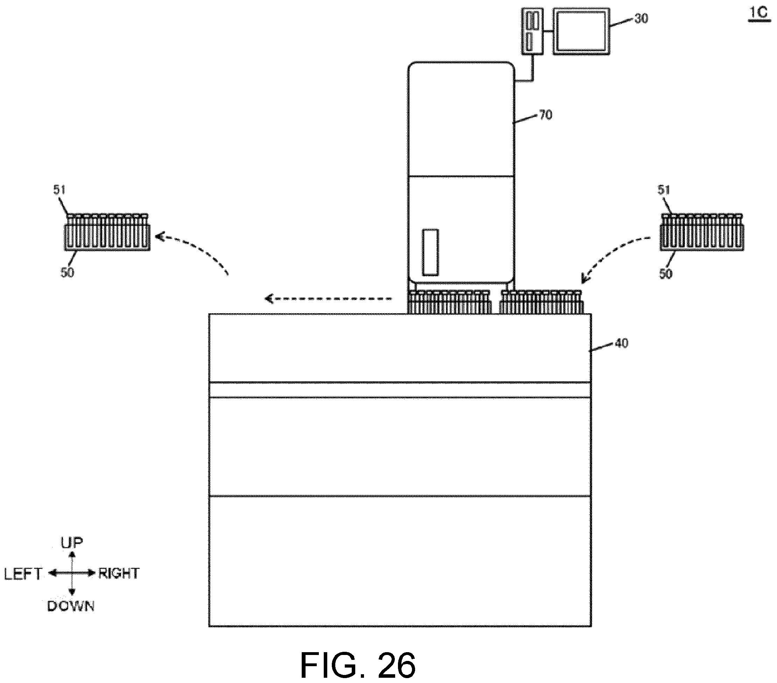

FIG. 26 is a front view schematically showing the configuration of a sample analyzer 1C as an example of the sample analyzer according to the third embodiment.

The sample analyzer 1C, compared to the second embodiment shown in FIG. 16, includes an integrated measurement unit 70 instead of the FCM measurement unit 10 and the GCM measurement unit 20.

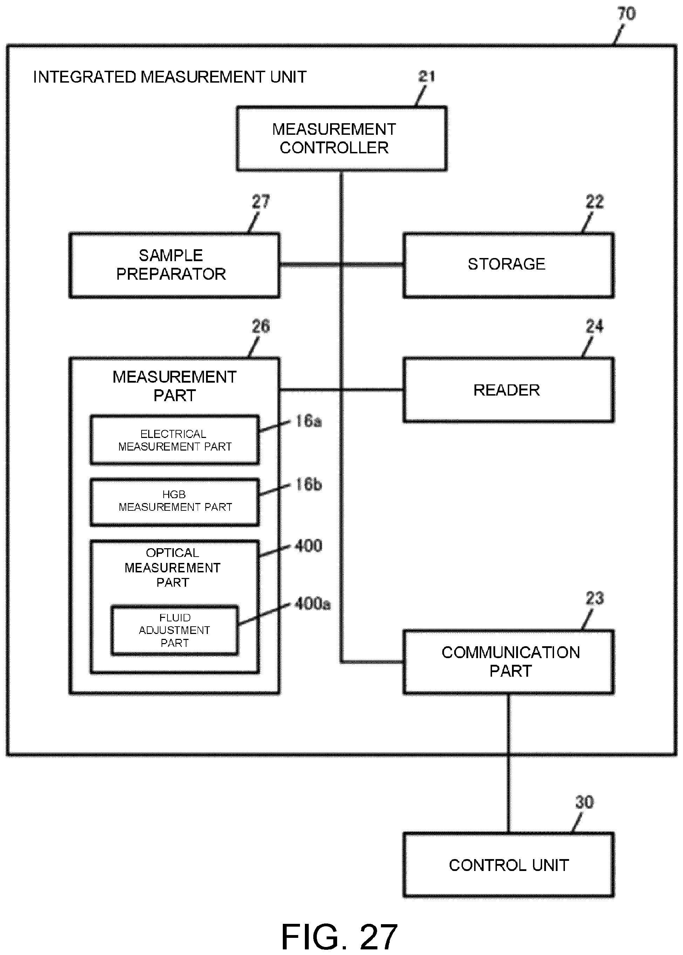

FIG. 27 is a block diagram showing an example of the functional configuration of the integrated measurement unit 70.

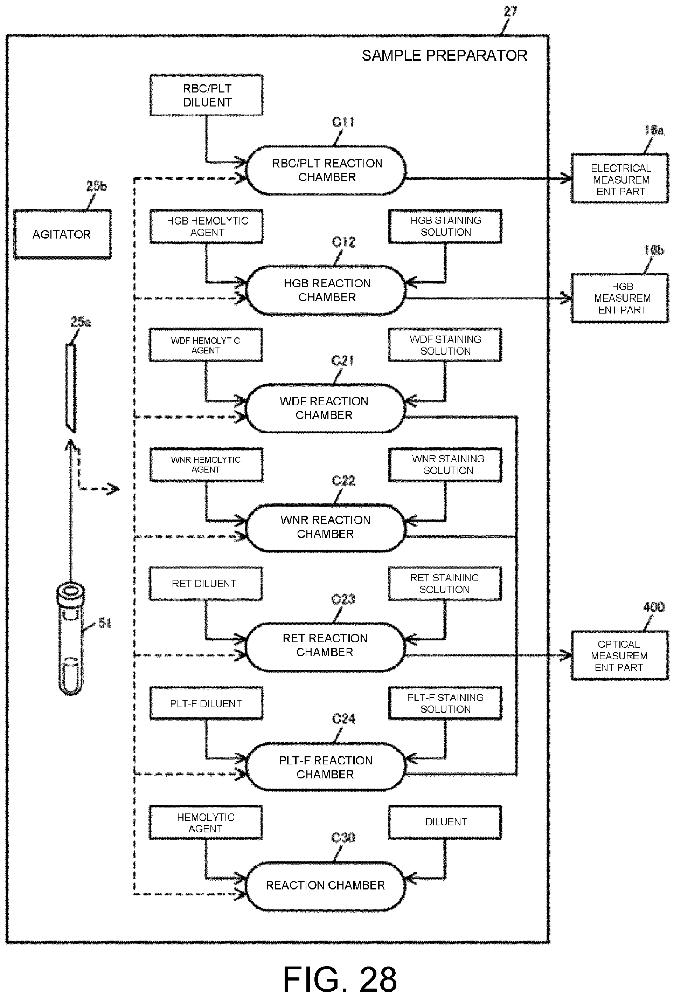

The integrated measurement unit 70, compared to the GCM measurement unit 20 of the first embodiment shown in FIG. 2, includes the electrical measurement part 16a and HGB measurement part 16b shown in FIG. 17, and includes a sample preparator 27 and an optical measurement part 400 instead of the sample preparator 25 and optical measurement part 200. The fluid adjustment part 400a in the optical measurement part 400 adjusts the flow rate per unit time of the measurement sample in the flow cell 201 of the optical measurement part 400 and is configured similarly to the fluid adjustment part 200a in FIG. 2. The sample preparator 27 will be described later with reference to FIG. 28. The optical system of the optical measurement part 400 will be described later with reference to FIG. 29.

The electrical measurement part 16a and HGB measurement part 16b perform signal processing on the detection signal obtained by measurement and output the processed measurement information to the measurement controller 21.

The optical measurement part 400 performs signal processing on the detection signal obtained by measurement and outputs the measurement data to the measurement controller 21.

The measurement controller 21 stores the measurement data output from the measurement part 26 in the storage 22. When the measurement of one sample is completed, the measurement controller 21 transmits the measurement data stored in the storage 22 to the control unit 30 in association with the sample ID read by the reader 24.

FIG. 28 is a block diagram showing an example of the functional configuration of the sample preparator 27.

The sample preparator 27, compared to the sample preparator 25 of the first embodiment shown in FIG. 3, includes the reaction chambers C11, C12, and C21-C24 shown in FIG. 18. The reaction chambers C11 and C12 are connected to the electrical measurement part 16a and HGB measurement part 16b, respectively, and the reaction chambers C21-C24 and C30 are connected to the optical measurement part 400.

The aspiration pipette 25b aspirates the sample from the sample container 51 agitated by the agitator 25a and dispenses the aspirated sample as appropriate into the reaction chambers C11, C12, C21-C24, and C30.

The measurement samples prepared in the reaction chambers C21-C24 and C30 are each individually flowed into the flow cell 201 and measured by the optical measurement part 400.

The optical measurement part 400 measures the measurement samples prepared in the reaction chambers C21-C24 to acquire detection signals, performs signal processing on the acquired detection signals, and obtains measurement data.
The optical measurement part 400 also measures the measurement sample prepared in the reaction chamber C30 to acquire a detection signal, performs signal processing on the acquired detection signal, and obtains a waveform signal.

FIG. 29 is a diagram schematically showing the configuration of the optical measurement part 400.

The optical measurement part 400, compared to the optical measurement part 200 of FIG. 4, includes the light source 111, collimator lens 112, cylindrical lens 113, beam stopper 122, optical filters 123, 132, and 142, and light detectors 124, 133, and 143 of the optical measurement part 100 shown in FIG. 19, and further includes dichroic mirrors 115, 125, 134, and 144, and a condenser lens 126.

The dichroic mirror 115 reflects light of wavelength λ10 from the light source 111 and transmits light of wavelength λ20 from the light source 211. The dichroic mirror 115 aligns the optical axis of the light from the light source 111 with the central axis of the light from the diffractive optical element 215. The condenser lens 216 condenses the light from the light sources 111 and 211 onto the flow path 201a of the flow cell 201. The condenser lens 216 is configured to suppress chromatic aberration for light of wavelengths λ10 and λ20.The beam spot BS (see FIG. 20) of the direct illumination light from the light source 111 is positioned at the center of the irradiation region R shown in FIG. 5. The diffracted illumination light from the light source 211 is irradiated onto the irradiation region R as in FIG. 5.

The collimator lens 112, cylindrical lens 113, dichroic mirror 115, and condenser lens 216 constitute an illumination optical system 206 that irradiates cells passing through the flow cell 201 with direct illumination light from the light source 111.

As in the second embodiment, when direct illumination light of wavelength λ10 is irradiated onto cells flowing through the flow cell 201, forward scattered light of wavelength λ10, side scattered light of wavelength λ10, and fluorescence of wavelength λ11 are generated from the portions of the cells irradiated with the light. When diffracted illumination light of wavelength λ20 is irradiated onto cells flowing through the flow cell 201, forward scattered light of wavelength λ20, side scattered light of wavelength λ20, and fluorescence of wavelength λ21 are generated from the portions of the cells irradiated with the light.

The dichroic mirror 125 reflects the direct illumination light and forward scattered light based on the direct illumination light, and transmits the diffracted illumination light and forward scattered light based on the diffracted illumination light. The direct illumination light and diffracted illumination light that have passed through the flow cell 201 are blocked by the beam stoppers 122 and 222, respectively. The condenser lens 126 condenses the forward scattered light based on the diffracted illumination light that has passed through the beam stopper 122 onto the light detector 124. The dichroic mirror 134 reflects the side scattered light based on the direct illumination light and transmits the side scattered light based on the diffracted illumination light. The dichroic mirror 144 reflects the fluorescence based on the direct illumination light and transmits the fluorescence based on the diffracted illumination light. The light detectors 124, 133, 143, 225, 233, and 243 receive the corresponding light and output detection signals.

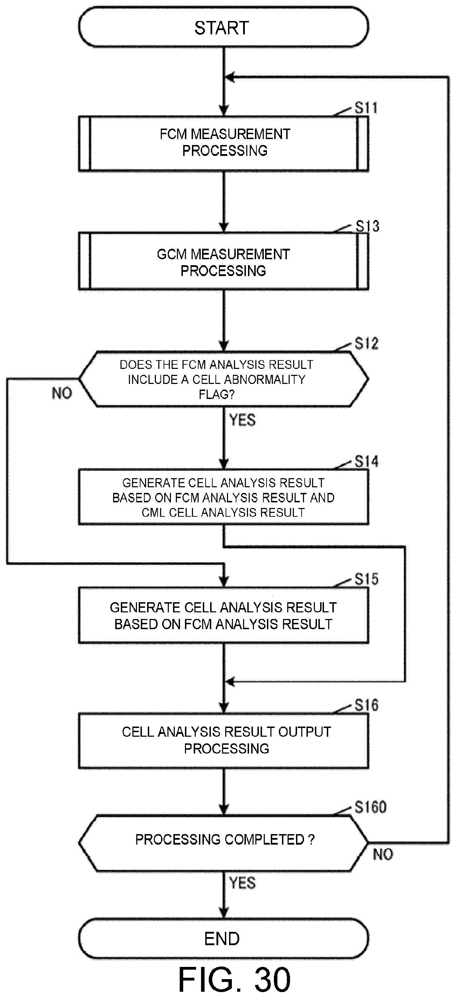

FIG. 30 is a flowchart showing an example of the flow of control processing related to measurement by the control unit 30.

The control processing in FIG. 30, compared to the second embodiment shown in FIG. 21, executes the GCM measurement processing of step S13 between steps S11 and S12. That is, in the third embodiment, the GCM measurement processing is executed regardless of the FCM analysis result. In the FCM measurement processing of the third embodiment, the integrated measurement unit 70 performs processing similar to that in FIG. 22, and in the GCM measurement processing, the integrated measurement unit 70 performs processing similar to steps S110 to S 140 in FIG. 10, for example.

### <Effects of the Third Embodiment>

According to this embodiment, in the sample analyzer (for example, sample analyzer 1C), the measurement part (for example, measurement part 26: optical measurement part 400) acquires information for identifying at least leukocytes among the cells contained in the sample. Then, the analysis part (for example, control unit 30) acquires the number (for example, CML cell count) or ratio (for example, CML cell content ratio) of leukemia cells among the leukocytes identified based on the information acquired by the measurement part, based on the optical information obtained by the measurement part.
Thus, the number or ratio of leukemia cells among the leukocytes identified based on the information for identifying at least leukocytes among the cells contained in the sample can be obtained based on the optical information.

Further, according to this embodiment, the measurement part acquires information for identifying at least granulocytes among the cells contained in the sample, and the analysis part acquires the number or ratio of leukemia cells among the granulocytes identified based on the information acquired by the measurement part, based on the optical information obtained by the measurement part.
Thus, the number or ratio of leukemia cells among the granulocytes identified based on the information for identifying at least granulocytes among the cells contained in the sample can be obtained based on the optical information.

In this case, the information for identifying at least granulocytes among the cells contained in the sample may include the intensity of scattered light obtained by irradiating the cells with a plurality of diffracted lights or a single illumination light.
Thus, the number or ratio of leukemia cells among the granulocytes identified based on information for identifying at least granulocytes among the cells contained in the sample, including the intensity of scattered light obtained by irradiating the cells with a plurality of diffracted lights or a single illumination light, can be obtained based on the optical information.

### <First Modification of the Third Embodiment>

In the third embodiment, as shown in FIG. 30, the first analysis result is determined in step S12, and either step S14 or S15 is executed according to the first analysis result. However, the determination in step S12 may be omitted.

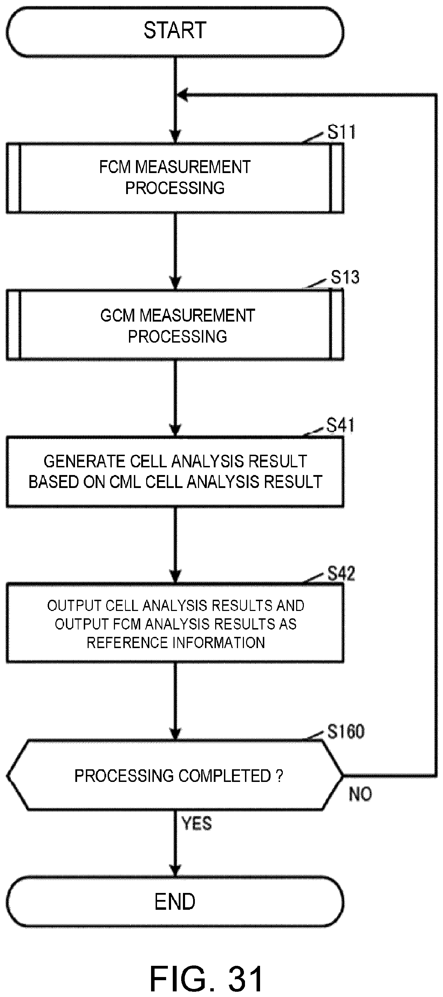

FIG. 31 is a flowchart showing an example of the flow of control processing related to measurement by the control unit 30 according to this modification.

In the control processing of this modification, compared to the flowchart shown in FIG. 30, steps S41 and S42 are added instead of steps S12 and S14-S16.

In step S41, the controller 31 of the control unit 30 generates cell analysis results based on the GCM analysis results. The cell analysis results in this case include the count values (e.g., total leukocyte count and CML cell count) and CML cell content ratio included in the GCM analysis results. In step S42, the controller 31 outputs (e.g., displays) the cell analysis results generated in step S41 to the cell analysis result screen 300, and also outputs (e.g., displays) the FCM analysis results obtained in the FCM measurement processing of step S11 as reference information. In this case, the FCM analysis results may be additionally displayed on the cell analysis result screen 300 when a button provided on the cell analysis result screen 300 is operated, or the FCM analysis results may be displayed together with a label indicating that they are reference information on the cell analysis result screen 300.

In steps S41 and S42 of FIG. 31, the cell analysis results are generated based on the GCM analysis results and the FCM analysis results are displayed as reference information, but the cell analysis results may be generated based on the FCM analysis results and the GCM analysis results may be displayed as reference information. For example, the control unit 30 may selectively determine whether to generate the cell analysis results based on the FCM analysis results or the GCM analysis results. For example, the control unit 30 may selectively determine whether to generate the cell analysis results based on the FCM analysis results or the GCM analysis results according to the settings or operations by the laboratory technician.

### <Second Modification of the Third Embodiment>

In the third embodiment, the measurement sample used in the GCM measurement processing is prepared in the reaction chamber C30, but is not limited thereto, and the RBC/PLT measurement sample prepared in the reaction chamber C11 may be used in the GCM measurement processing.

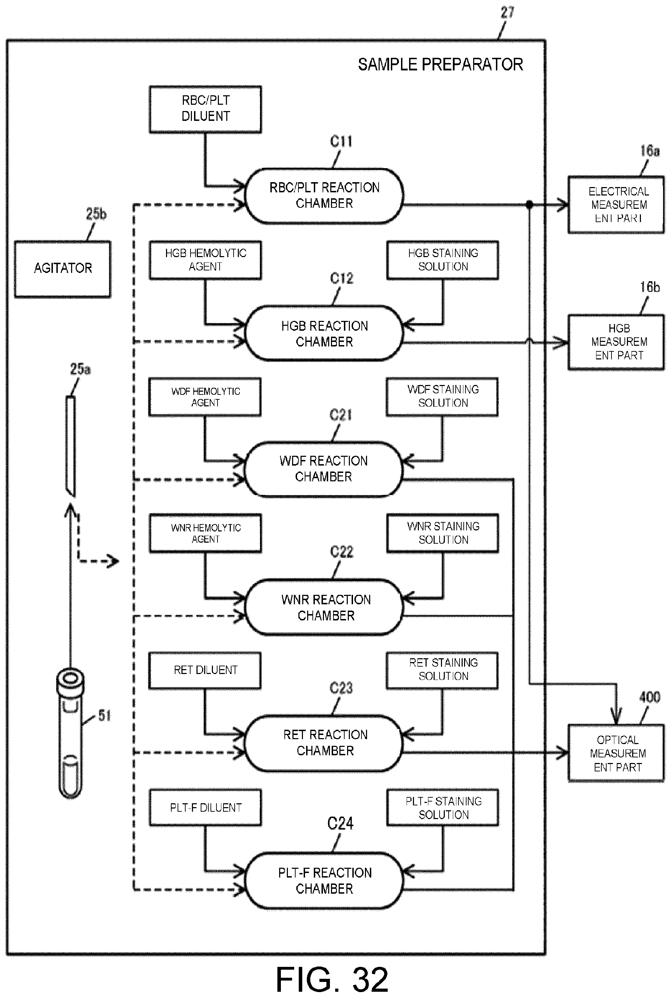

FIG. 32 is a block diagram showing the functional configuration of the sample preparator 27 according to this modification.

In the sample preparator 27 of this modification, compared to the third embodiment shown in FIG. 28, the reaction chamber C30 is omitted, and the reaction chamber C11 and the optical measurement part 400 are connected. In this modification, regarding the second measurement sample, the RBC/PLT measurement sample prepared in the reaction chamber C11 is flowed into the flow cell 201, the diffracted illumination light is irradiated onto the measurement sample, and the waveform signal is acquired. In this case, since fluorescence based on the diffracted illumination light is not acquired, the GCM analysis result is obtained by analyzing the waveform signal based on the forward scattered light and side scattered light based on the diffracted illumination light.

According to this modification, the reaction chamber C30 can be omitted, so the configuration of the sample analyzer 1C can be simplified.

In this modification, in the GCM measurement processing, the waveform signal was acquired by flowing the RBC/PLT measurement sample into the flow cell 201, but the waveform signal may be acquired by flowing the WDF measurement sample into the flow cell 201.

In this case, the GCM measurement processing may be performed simultaneously with the FCM measurement processing. That is, when the WDF measurement sample prepared in the reaction chamber C21 is flowed into the flow cell 201, information for identifying leukocytes and the waveform signal may be acquired simultaneously. However, in this case, in order to properly acquire the waveform signal, it is necessary to reduce the flow rate per unit time of the WDF measurement sample flowing through the flow cell 201 by controlling the fluid adjustment part 400a, compared to the case where only information for identifying leukocytes is acquired from the WDF measurement sample. However, since information for identifying leukocytes and the waveform signal can be acquired simultaneously, the throughput of sample analysis can also be increased.

### <Fourth Embodiment>

As a standard treatment for CML, the introduction of TKIs, which are molecular targeted drugs as described above, has dramatically improved the prognosis of CML. However, many patients need to take TKIs for a long period, and various adverse events associated with long-term administration have been reported. As a treatment goal for CML, it is important to aim for treatment-free remission (TFR), in which remission is maintained even after discontinuation of TKI, but in reality, only about 50% of patients can achieve TFR.

The causative gene of CML, BCR::ABL1, is measured by quantitative PCR, and it has been reported that an early rapid decrease in BCR::ABL1 after initiation of TKI correlates with an increased likelihood of achieving TFR (Shanmuganathan N, et al, Early BCR-ABL1 kinetics are predictive of subsequent achievement of treatment-free remission in chronic myeloid leukemia, Blood. 2021, DOI: 10.1182/blood.2020005514). That is, predicting TKI treatment responsiveness in CML treatment is considered useful information for the attending physician in deciding whether to select or discontinue TKI.

This embodiment relates to predicting (inferring) the treatment responsiveness of CML based on the discrimination results of CML patient leukocytes by GCM at the time of initial diagnosis.

The contents described in the fourth embodiment can be similarly applied to any of the other embodiments and modifications.

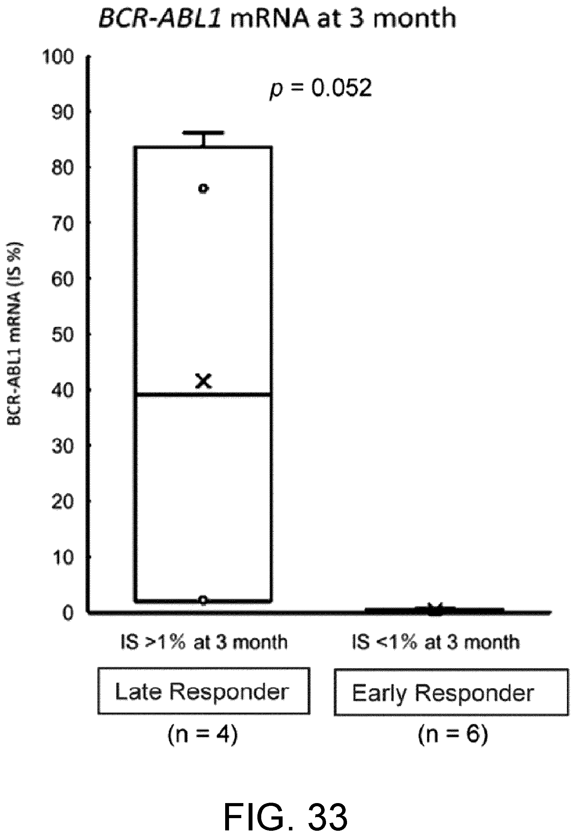

FIG. 33 shows a graph statistically comparing the IS% distribution between patients (n=4, "late responders") whose BCR::ABL1 mRNA (IS%) was 1% or more at 3 months of treatment (i.e., low drug efficacy) and patients (n=6, "early responders") whose BCR::ABL1 mRNA (IS%) was less than 1% at 3 months of treatment (i.e., high drug efficacy) among 10 cases of CML patients treated with second-generation TKI. The left graph corresponds to "late responders" (n=4), and the right graph corresponds to "early responders" (n=6). The vertical axis is (IS%).

This graph shows that at 3 months of treatment, there is a significant difference in BCR::ABL1 mRNA (IS%) values between late responders and early responders. As mentioned above, since an early rapid decrease in BCR::ABL1 after TKI initiation has been reported to correlate with a higher likelihood of achieving TFR, it is important to assume that early responders are patients with a high likelihood of achieving TFR and late responders are patients for whom achieving TFR is difficult, and to develop a treatment plan accordingly.

Late responders and early responders can be distinguished by tracking time-series data using the above PCR method, but at the time of initial diagnosis, the value of BCR::ABL1 mRNA (IS%) is around 100% in 10 cases of CML patients, and it is not possible to distinguish between them at this point. Therefore, the inventors considered a method for distinguishing between late responders and early responders even at the time of initial diagnosis. In this method, a classification model is generated (constructed) that classifies cells contained in a sample collected from a patient with chronic myeloid leukemia before the start of treatment (hereinafter referred to as "target patient") as CML cells and cells contained in a sample collected from a healthy subject as normal cells, and by using an index relating to the classification performance of CML cells and normal cells by this classification model, it is determined whether the target patient is a late responder (group with low therapeutic efficacy) or an early responder (group with high therapeutic efficacy). As the index, any index representing the classification performance of the classification model may be used, such as F1 score or AUC. The higher the classification performance, the more significant the morphological difference between CML cells and normal cells contained in the sample from the target patient.

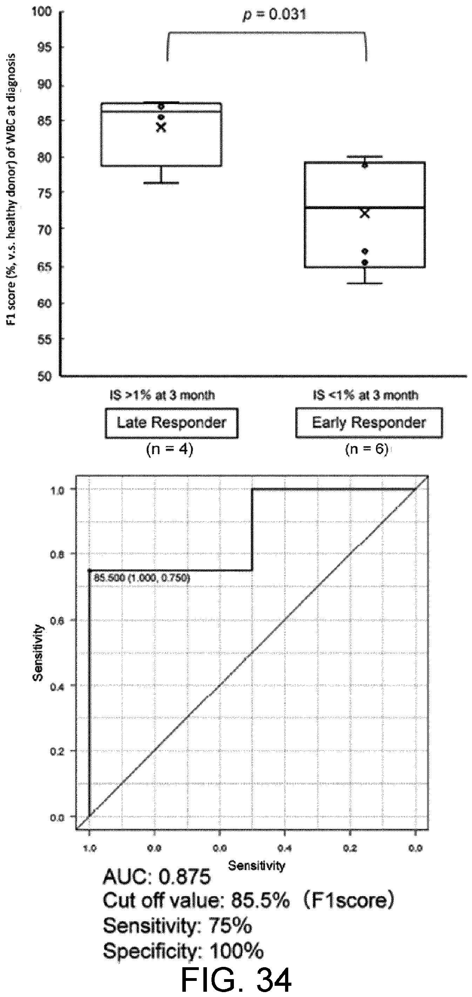

The upper part of FIG. 34 shows a graph comparing the F1 scores when CML cells and normal cells are classified by an AI algorithm trained using samples from CML patients, between late responders and early responders as shown in FIG. 33. The left graph corresponds to "late responders" (n=4), and the right graph corresponds to "early responders" (n=6). The vertical axis is the F1 score, which is the discrimination result when comparing CML patient samples and healthy subject samples by ghost cytometry. In other words, the vertical axis represents how much morphological difference there is between cells in CML patient samples and cells in healthy subject samples.
This graph shows that late responders have a significantly higher F1 score than early responders.

The lower part of FIG. 34 shows the ROC curve when classifying late responders and early responders based on the F1 score.
As a result of this ROC analysis, when the cutoff value was set to "85.5%", it was found that the sensitivity was "75%" and the specificity was "100%".

That is, by focusing on the F1 score of the discrimination result using peripheral blood leukocytes from CML patients at initial diagnosis and peripheral blood leukocytes from healthy subjects, it may be possible to distinguish between late responders and early responders based on the CML cell content ratio at initial diagnosis.

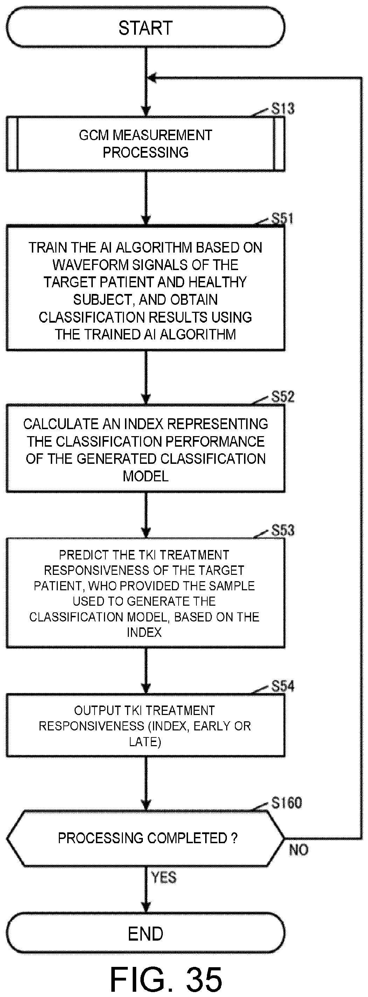

FIG. 35 is a flowchart showing an example of the flow of control processing related to measurement by the control unit 30. In this embodiment, for example, the control unit 30 in the sample analyzer 1 described in the various embodiments above may perform the following processing.

In step S13, the controller 31 controls the GCM measurement unit 20 so that GCM measurement processing is performed on the sample from the target patient. As a result, the controller 31 acquires waveform signals from the CML cells of the target patient in the GCM measurement unit 20.

In step S51, the controller 31 trains the AI algorithm 61 using the waveform signals of a portion of the cells (for example, 75% of all cells) among the waveform signals of multiple cells contained in the sample from the target patient as training data, that is, as "CML (+) signals." Furthermore, the controller 31 trains the AI algorithm 61 using the waveform signals of a portion of the cells (for example, 75% of all cells) among the waveform signals of multiple cells contained in the sample from one or more healthy subjects as training data, that is, as "CML (-) signals." As a result, the controller 31 obtains the trained AI algorithm 62.
The controller 31 inputs, into the trained AI algorithm 62, the waveform signals among the plurality of waveform signals obtained from the sample of the target patient that were not used as training data (for example, the remaining 25% of the cell waveform signals), and causes the AI algorithm 62 to classify the signals as positive or negative for CML cells. In this case, when the AI algorithm 62 classifies a signal as positive, it is counted as TP (True Positive), and when classified as negative, it is counted as FN (False Negative). Similarly, the controller 31 inputs, into the trained AI algorithm 62, the waveform signals among the plurality of waveform signals obtained from the sample of a healthy subject that were not used as training data, and causes the AI algorithm 62 to classify the signals as positive or negative for CML cells. In this case, when the AI algorithm 62 classifies a signal as positive, it is counted as FP (False Positive), and when classified as negative, it is counted as TN (True Negative).

Subsequently, in step S52, the controller 31 calculates an index (for example, F1 score) representing the classification performance of the classification model based on the classification results by the AI algorithm 62, that is, TP, FN, FP, and TN.

In step S53, the controller 31 predicts the TKI treatment responsiveness of the target patient (whether the target patient is an early responder or a late responder) based on the calculated index (for example, by comparing the F1 score with a threshold (cutoff value)).

Then, in step S54, the controller 31 outputs (for example, displays) a cell analysis result screen including the predicted TKI treatment responsiveness to the display 34. The TKI treatment responsiveness may include, for example, the F1 score.

### <Effects of the Fourth Embodiment>

According to the method of this embodiment, a plurality of diffracted lights generated by incident light on a diffractive optical element are irradiated onto cells contained in a sample to acquire optical information of the cells, and the sample includes a first sample collected from a patient with chronic myeloid leukemia before the start of treatment (for example, a sample collected from a patient with chronic myeloid leukemia (target patient) before the start of treatment) and a second sample collected from a healthy subject. Based on the optical information obtained from the first and second samples, a classification model for classifying leukemia cells is generated, an index relating to the classification performance of leukemia cells and normal cells by the generated classification model is obtained, and information regarding the efficacy of a therapeutic agent for chronic myeloid leukemia for the patient is output based on the index.
By using the optical information obtained from the first sample collected from a patient with chronic myeloid leukemia before the start of treatment and the second sample collected from a healthy subject, a classification model for classifying leukemia cells can be easily generated. Then, by obtaining an index relating to the classification performance of leukemia cells and normal cells by the generated classification model and outputting information regarding the efficacy of a therapeutic agent for chronic myeloid leukemia for the above-mentioned patient with chronic myeloid leukemia before the start of treatment based on the obtained index, it can be made available for confirmation by the attending physician or the like.

In this case, the therapeutic agent for chronic myeloid leukemia may be a tyrosine kinase inhibitor (TKI).

### <Modification of the Fourth Embodiment>

As the therapeutic agent for chronic myeloid leukemia, a tyrosine kinase inhibitor, which is a type of molecular targeted drug, has been exemplified, but a molecular targeted drug other than a tyrosine kinase inhibitor may also be used. In addition, although molecular targeted therapy is often used as standard treatment at present, other treatment methods may be applied, and the therapeutic agent selected for such treatment methods may also be used.

### <Other Embodiments>

In the above embodiments, a single direct illumination light having a single beam spot BS is irradiated onto cells flowing through the flow cell, but a plurality of direct illumination lights each having a single beam spot may be irradiated onto cells flowing through the flow cell.
That is, in the optical measurement part 100 shown in FIG. 19, another direct illumination light having a single beam spot based on light from another light source may be irradiated onto cells flowing through the flow cell 101. Also, in the optical measurement part 400 shown in FIG. 29, another direct illumination light having a single beam spot based on light from another light source may be irradiated onto cells flowing through the flow cell 201. The wavelength of the light irradiated from the other light source is preferably different from the wavelength of the light irradiated from the light source 111 or light source 211.

In the above embodiments, the diffractive optical element 215 may have a condensing function. In this case, for example, the diffraction pattern formed on the diffractive optical element 215 itself may have the condensing function, a diffraction pattern for generating diffracted light may be formed on the incident surface of the diffractive optical element 215, and a pattern having a lens function or a fresnel lens may be formed on the exit surface of the diffractive optical element 215. Further, if the diffractive optical element 215 has the condensing function, the condenser lens 216 may be omitted.

In the above embodiments, the diffractive optical element 215 is a transmission-type diffractive optical element, but it may be a reflection-type diffractive optical element.

In the above embodiments, the arithmetic unit 32 of the control unit 30 classifies cells using the AI algorithm 62 based on the detection signals from the light detectors 225, 233, and 243, but the invention is not limited thereto, and cells may be classified by comparing the pattern of the detection signals from the light detectors 225, 233, and 243 with a pattern stored in advance in the storage 33.

In the above embodiments, in the GCM measurement processing, count values and abnormal cell flags for CML cells were obtained, but count values and abnormal cell flags for cells other than CML cells, such as neutrophils, normal lymphocytes, monocytes, eosinophils, basophils, blasts, abnormal lymphocytes, atypical lymphocytes, immature granulocytes, and nucleated red blood cells, may also be obtained.

In the above embodiments, the sample was blood, but the invention is not limited thereto, and other body fluids may be used.

In the above embodiments, when the FCM analysis result meets the predetermined condition shown in step S12, the GCM measurement processing is executed, but the invention is not limited thereto, and the GCM measurement processing may be executed regardless of the FCM analysis result.

In the above embodiments, when the FCM analysis result meets the predetermined condition shown in step S12, the controller 31 of the control unit 30 may place the sample container 51 measured by the FCM measurement unit 10 at the sample intake position (sample supply position) of the GCM measurement unit 20, and when the FCM analysis result does not meet the predetermined condition, the conveyer 40 may be controlled so that the sample container 51 passes through the GCM measurement unit 20.

The embodiments of the present invention can be variously modified as appropriate within the scope of the technical idea described in the claims.

### [Remarks]

A specimen analysis apparatus comprising:
a measurement unit configured to acquire optical information of cells contained in a specimen by irradiating the cells with a plurality of diffracted lights generated by causing light to be incident on a diffractive optical element; and
an analysis unit configured to analyze the optical information obtained by the measurement unit using an artificial intelligence algorithm to acquire first information on leukemic cells contained in the specimen.

The specimen analysis apparatus according to item 1, wherein the analysis unit acquires a ratio of leukemic cells to leukocytes as the first information on leukemic cells.

The specimen analysis apparatus according to item 1, wherein the analysis unit acquires a number of leukemic cells as the first information on leukemic cells.

The specimen analysis apparatus according to item 1, further comprising a sample preparation unit configured to prepare a measurement sample by mixing a specimen and a reagent, wherein the sample preparation unit prepares a measurement sample in which red blood cells contained in the specimen are hemolyzed by using a hemolytic agent as the reagent.

The specimen analysis apparatus according to item 4, wherein the reagent does not contain a staining agent.

The specimen analysis apparatus according to item 1, wherein the measurement unit is configured to acquire second information that specifies at least leukocytes among the cells contained in the specimen, and the analysis unit acquires, based on the optical information, a number or a ratio of leukemic cells among the specified leukocytes.

The specimen analysis apparatus according to item 1, wherein the measurement unit is configured to acquire second information that specifies at least granulocytes among the cells contained in the specimen, and the analysis unit acquires, based on the optical information, a number or a ratio of leukemic cells among the specified granulocytes.

The specimen analysis apparatus according to item 6 or 7, wherein the second information includes an intensity of scattered light obtained by irradiating a cell with a single light.

The specimen analysis apparatus according to item 1, wherein the artificial intelligence algorithm has been trained with optical information of leukocytes contained in a specimen collected from a patient with chronic myeloid leukemia as training data.

The specimen analysis apparatus according to item 1, wherein the artificial intelligence algorithm has been trained with optical information of granulocytes contained in a specimen collected from a patient with chronic myeloid leukemia as training data.

The specimen analysis apparatus according to item 1, further comprising a first measurement unit that acquires a measurement result regarding information on blood cells contained in a specimen,
wherein the measurement unit is a second measurement unit different from the first measurement unit, and the second measurement unit executes measurement of the specimen to acquire optical information of cells contained in the specimen in response to the measurement result of the specimen by the first measurement unit satisfying a predetermined condition.

The specimen analysis apparatus according to item 11, wherein the predetermined condition is satisfied by an increase of a specific type of leukocyte or an appearance of blasts.

The specimen analysis apparatus according to item 12, wherein the specific type of leukocyte is a basophil.

The specimen analysis apparatus according to item 1, wherein the artificial intelligence algorithm is a model trained using specimens in which a ratio of BCR::ABL fusion positive leukocytes among leukocytes is greater than or equal to a predetermined value.

The specimen analysis apparatus according to item 1, wherein the artificial intelligence algorithm is a model trained using specimens which is determined to have Major BCR::ABL1 mRNA (%) at 80% or more by PCR testing.

The specimen analysis apparatus according to item 1, wherein the measurement unit includes a flow cell, and wherein the measurement unit is configured to cause the cells to flow through the flow cell and irradiate the plurality of diffracted lights to the cells flowing in the flow cell.

The specimen analysis apparatus according to item 1, wherein the specimen is blood specimen.

A specimen analysis method comprising:
acquiring optical information of cells contained in a specimen by irradiating the cells with a plurality of diffracted lights generated by causing light to be incident on a diffractive optical element; and
analyzing the optical information using an artificial intelligence algorithm to acquire information on leukemic cells contained in the specimen.

A method comprising:
acquiring optical information of cells contained in a specimen by irradiating the cells with a plurality of diffracted lights generated by causing light to be incident on a diffractive optical element, wherein the specimen includes a first specimen collected from a patient with chronic myeloid leukemia before the start of treatment and a second specimen collected from a healthy individual;
generating a classification model configured to classify leukemic cells based on the optical information obtained from the first and second specimens;
acquiring an index regarding a performance of classification between leukemic cells and normal cells by the generated classification model; and
outputting information regarding a therapeutic efficacy of a therapeutic drug for chronic myeloid leukemia for the patient based on the index.

The method according to item 19, wherein the therapeutic drug is a tyrosine kinase inhibitor.

## Claims

1. A specimen analysis apparatus (1A, 1B, 1C, 1) comprising:
a measurement unit (20) configured to acquire optical information of cells contained in a specimen by irradiating the cells with a plurality of diffracted lights generated by causing light to be incident on a diffractive optical element (215); and
an analysis unit (30) configured to analyze the optical information obtained by the measurement unit (20) using an artificial intelligence algorithm to acquire first information on leukemic cells contained in the specimen.

2. The specimen analysis apparatus (1A, 1B, 1C, 1) according to claim 1, wherein the analysis unit (30) acquires a ratio of leukemic cells to leukocytes as the first information on leukemic cells.

3. The specimen analysis apparatus (1A, 1B, 1C, 1) according to claim 1, wherein the analysis unit (30) acquires a number of leukemic cells as the first information on leukemic cells.

4. The specimen analysis apparatus (1A, 1B, 1C, 1) according to any one of claims 1 to 3, further comprising a sample preparation unit (25) configured to prepare a measurement sample by mixing a specimen and a reagent, wherein the sample preparation unit (25) prepares the measurement sample in which red blood cells contained in the specimen are hemolyzed by using a hemolytic agent as the reagent.

5. The specimen analysis apparatus (1A, 1B, 1C, 1) according to claim 4, wherein the reagent does not contain a staining agent.

6. The specimen analysis apparatus(1A, 1B, 1C, 1) according to any one of claims 1 to 5, wherein the measurement unit (20) is configured to acquire second information that specifies at least leukocytes among the cells contained in the specimen, and the analysis unit (30) acquires, based on the optical information, a number or a ratio of leukemic cells among the specified leukocytes.

7. The specimen analysis apparatus (1A, 1B, 1C, 1) according to any one of claims 1 to 6, wherein the measurement unit (20) is configured to acquire second information that specifies at least granulocytes among the cells contained in the specimen, and the analysis unit (30) acquires, based on the optical information, a number or a ratio of leukemic cells among the specified granulocytes.

8. The specimen analysis apparatus (1A, 1B, 1C, 1) according to claim 6 or 7, wherein the second information includes an intensity of scattered light obtained by irradiating a cell with a single light.

9. The specimen analysis apparatus (1A, 1B, 1C, 1) according to any one of claims 1 to 8, wherein the artificial intelligence algorithm has been trained with optical information of leukocytes contained in a specimen collected from a patient with chronic myeloid leukemia as training data.

10. The specimen analysis apparatus (1A, 1B, 1C, 1) according to any one of claims 1 to 9, wherein the artificial intelligence algorithm has been trained with optical information of granulocytes contained in a specimen collected from a patient with chronic myeloid leukemia as training data.

11. The specimen analysis apparatus (1A, 1B, 1C, 1) according to any one of claims 1 to 10, further comprising a first measurement unit (10) that acquires a measurement result regarding information on blood cells contained in a specimen,
wherein the measurement unit (20) is a second measurement unit (20) different from the first measurement unit (10), and the second measurement unit (20) executes measurement of the specimen to acquire optical information of cells contained in the specimen in response to the measurement result of the specimen by the first measurement unit (10) satisfying a predetermined condition.

12. The specimen analysis apparatus (1A, 1B, 1C, 1) according to claim 11, wherein the predetermined condition is satisfied by an increase of a specific type of leukocyte or an appearance of blasts.

13. The specimen analysis apparatus (1A, 1B, 1C, 1) according to claim 12, wherein the specific type of leukocyte is a basophil.

14. A sample analysis method comprising:
acquiring optical information of cells contained in a specimen by irradiating the cells with a plurality of diffracted lights generated by causing light to be incident on a diffractive optical element (215), wherein the specimen includes a first specimen collected from a patient with chronic myeloid leukemia before the start of treatment and a second specimen collected from a healthy individual;
generating a classification model (62) configured to classify leukemic cells based on the optical information obtained from the first and second specimens;
acquiring an index regarding a performance of classification between leukemic cells and normal cells by the generated classification model (62); and
outputting information regarding a therapeutic efficacy of a therapeutic drug for chronic myeloid leukemia for the patient based on the index.

15. The sample analysis method according to claim 14, wherein the therapeutic drug is a tyrosine kinase inhibitor.
